# EUROPEAN PATENT SPECIFICATION

(11) **EP 1 658 503 B1**
(45) Date of publication and mention of the grant of the patent: **18.11.2009**
(21) Application number: 04768216.6
(22) Date of filing: 26.08.2004
(51) Int. Cl.: G01N 33/58, C07D 219/00, C09K 11/00, C09B 15/00

(54) **CHEMILUMINESCENT COMPOUNDS**
CHEMILUMINESZENZVERBINDUNGEN
COMPOSES CHIMIOLUMINESCENTS

(30) Priority: 29.08.2003 GB 0320236
(43) Date of publication of application: 24.05.2006
(73) Proprietor: Molecular Light Technology Research Limited, Cardiff CF14 5DL (GB)
(72) Inventor: WEEKS, Ian, Cardiff CF23 6NE (GB); RUTTER, Andrew, James, Cardiff CF14 3PZ (GB); LI, Zhaoqiang, Reading RG6 3DB (GB); SMITH, Keith, Swansea SA3 5DT (GB)
(74) Representative: Lambert, Ian Robert
(86) International application number: PCT/GB2004/003660
(87) International publication number: WO 2005/022161

(56) References cited:
- US-A- 5 283 334
- US-A- 5 438 139
- US-A- 5 750 698
- SATO N: "Synthesis and Properties of New Luminescent 10-Carboxymethylacridinium Derivatives" TETRAHEDRON LETTERS, ELSEVIER SCIENCE PUBLISHERS, AMSTERDAM, NL, vol. 37, no. 47, 18 November 1996 (1996-11-18), pages 8519-8522, XP004068705 ISSN: 0040-4039
- SATO HIROSHI ET AL: "Competitive chemiluminescent immunoassay for estradiol using an N-functionalized acridinium ester" JOURNAL OF BIOLUMINESCENCE AND CHEMILUMINESCENCE, vol. 11, no. 1, 1996, pages 23-29, XP009041434 ISSN: 0884-3996
- RAZAVI ZIA ET AL: "Stable and versatile active acridinium esters I" LUMINESCENCE (CHICHESTER), vol. 15, no. 4, July 2000 (2000-07), pages 239-244, XP009041418 ISSN: 1522-7235
- ZOMER G ET AL: "CHEMILUMINOGENIC LABELS OLD AND NEW" ANALYTICA CHIMICA ACTA, vol. 227, no. 1, 1989, pages 11-20, XP009041398 & 3RD INTERNATIONAL SYMPOSIUM ON QUANTITATIVE LUMINESCENCE SPECTROMETRY IN BIOMEDICAL SCIENCES, GENT, ISSN: 0003-2670
- WILSON R ET AL: "Electrochemiluminescence determination of 2',6'-difluorophenyl 10-methylacridan-9-carboxylate" ANALYTICAL CHEMISTRY, AMERICAN CHEMICAL SOCIETY. COLUMBUS, US, vol. 73, no. 4, 15 February 2001 (2001-02-15), pages 763-767, XP002267753 ISSN: 0003-2700

## Description

The present invention relates to compounds which are of use as chemiluminescent labels in a variety of ligand binding assays, to assay methods employing these compounds and to kits including them. In particular, the invention relates to certain acridinium derivatives.

Chemiluminescence is the phenomenon whereby a chemical reaction leads to the emission of light. This occurs because the reaction product is formed in an electronically excited state and it therefore emits electromagnetic radiation (often in the visible spectrum) in order to return to its ground state.

The use of chemiluminescence energy transfer systems for the labelling of assay reagents has been described previously. In such labelling systems, there is provided a light emitting donor-acceptor pair the relative distance between which is dependent on the presence or absence of a ligand-binding event. For example, a ligand is coupled to a chemiluminescent emitter and the corresponding anti-ligand is coupled to an energy acceptor such that when the ligand and anti-ligand are present as a bound complex, the chemiluminescent emission is modulated by the close proximity of the acceptor. Such modulation is manifest either as a loss of chemiluminescence intensity overall or as a change in wavelength of the emission depending on the nature of the acceptor molecule.

Chemiluminescent acridinium salts have been widely used as labelling molecules in ligand binding assays such as immunoassays and nucleic acid hybridisation assays. Such assays involve coupling these molecules to the ligand or anti-ligand either directly by covalent bonds or by secondary ligand binding such as is exemplified by biotin-streptavidin binding where the labelling molecule is coupled to the streptavidin.

The features required by molecules capable of acting as chemiluminescent acridinium labelling molecules are simply that 1) they are capable of coupling to (i.e. labelling) the ligand without affecting the reactivity of the said ligand and 2) the labelling compound substantially retains its chemiluminescent activity. Any particular molecular structure that achieves these features is capable of acting as a labelling compound and, indeed, many such structures have been described.

For example, EP-A-0082636 relates to acridinium compounds as chemiluminescent labels of the formula 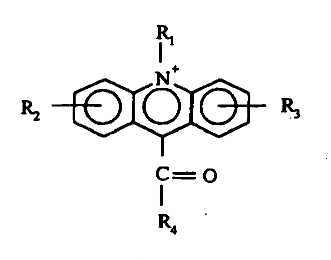
in which R₁ represents H, C₁-C₁₀ optionally substituted alkyl, alkenyl alkynyl or aryl;
R₂ and R₃ are hydrogen, amino, substituted amino, carboxyl, hydroxyl alkoxyl, nitro or halo; and
R₄ is an optionally substituted phenoxy moiety.

Acridinium compounds of this type emit chemiluminescent radiation on treatment with basic hydrogen peroxide which oxidises the acridinium system and leads *via* a dioxetanone intermediate to the formation of a ketone group at the C-9 position. This product is formed in an excited state which subsequently relaxes to its corresponding ground state with emission of electromagnetic radiation.

The compounds described in EP-A-0082636, however, bind to the ligand *via* a substituent of the phenoxy moiety R₄ and therefore after treatment with hydrogen peroxide, the radiation emitting acridone ring system is no longer bound to the ligand. This may have certain advantages in that the chemiluminescent radiation cannot be quenched non-specifically by the ligand but, on the other hand, the use of this type of compound may not be optimal in a chemiluminescence energy transfer system where specific quenching is required.

This potential problem was discussed by Patel and Campbell in Clin. Chem., 29(9), 1604-1608 (1983). This document relates to a chemiluminescence energy transfer assay using the chemiluminescent compound aminobutylethyl-isoluminol and its isothiocyanate derivative. The authors state that attempts to improve the magnitude of the energy transfer using different chemiluminescent donors have failed. In particular they state "Nor has any energy transfer yet been detected between acridinium-labelled IgG...". They speculate that this could be because during the chemiluminescent reaction the excited N-methyl acridone is cleaved from the antigen. The authors also point out that the acridinium excitation reaction is best catalysed at high pH, which would disrupt the antibody-antigen complex.

EP-A-0324202 relates to chemiluminescent acridinium compounds of the formula: 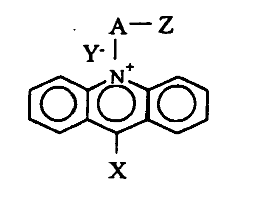
in which A is a divalent organic moiety;
X is a substituent which can form a dioxetane together with the C-9 acridinium atom and hydrogen peroxide;
Y is a counter ion; and
Z is a functional group;
and wherein the benzene rings may carry one or more substituents, such as lower alkyl, lower alkoxy, halogen etc.

US 5,438,139 discloses acridinium compounds of general formula: 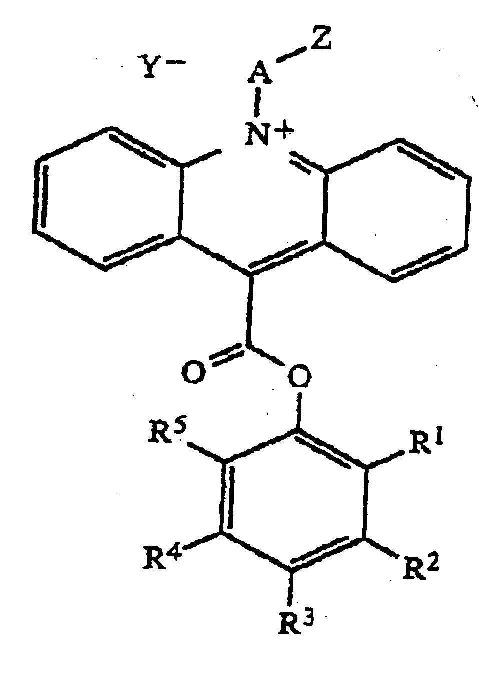 wherein A is a linker having from 1-4 carbon atoms, Z is a binding group having activity for binding with specific ligands, and R1 is selected from halogen, alkyl or aryl groups, and R2-R5 are each individually selected from a hydrogen atom, an alkyl group, an aryl group, an alkoxy group, an acetyl group, a nitro group, a halogen atom or a carbonyl group, for use as chemiluminescent labelling agents.

The difference between these compounds and those of EP-A-0082636 is said to be that they bind to a ligand *via* the group Z. Therefore, following the reaction with hydrogen peroxide, the chemiluminescent emitter remains attached to the ligand.

However, the present inventors have come to realise that the problems associated with this technology are more complex than previously appreciated and that contrary to the teaching of EP-A-0324202, most of the compounds to which it relates are not suitable for use in chemiluminescence energy transfer systems.

Indeed, such have been the problems with chemiluminescence energy transfer systems that, while energy transfer systems using fluorescence emitting labels are widely used, their chemiluminescent counterparts have not found commercial application. This is in spite of the fact that chemiluminescence can be detected with far greater sensitivity than conventional fluorescence thus making it far more desirable as an analytical end-point.

One reason for the success of fluorescent systems and the lack of success of their chemiluminescent counterparts in resonance energy transfer systems is that initiation of fluorescence is a physical, non-invasive process whereas initiation of chemiluminescence requires a change in chemical conditions which may both alter the optical properties of the acceptor and disrupt the very ligand/anti-ligand interaction which it is designed to monitor.

Although the acridinium compounds described in EP-A-0324202 are purported to be useful in energy transfer assays, the present inventors have come to realise that most of these compounds are not capable of permitting the chemiluminescent reaction to be initiated whilst maintaining the integrity of the binding complex being evaluated. This is certainly true of the sole example of an acridinium ester labelling compound, in which the group X is a phenoxycarbonyl group (see Examples 21 and 25).

The problem arises because most of the compounds of EP-A-0324202, including the only acridinium ester compound exemplified, do not yield useful chemiluminescent emission except under the extreme alkaline conditions set forth in the only example, in which chemiluminescence is initiated at pH ≥ 12. However, most binding complexes are dissociated at the extremes of alkaline pH required to initiate the chemiluminescent reaction of the labelling molecules. This means that the use of many of the acridinium compounds of EP-A-0324202 as chemiluminescent labels is very limited since they cannot workably be used in chemiluminescent resonance energy transfer systems which universally require the integrity of the binding complex to be maintained at the time of signal measurement. This was clearly not appreciated by the authors of EP-A-0324202 since the document does not mention this consideration. There is therefore no way of establishing from the teaching of EP-A-0324202 which compounds would in fact be workable as purported.

Another problem of the high pH values needed with prior art compounds is that the absorption spectrum of many of the commonly used quencher molecules is pH dependent and the elevated pH required for initiation of chemiluminescence from conventional acridinium labels renders such quenchers useless. Again, this problem was not appreciated by the authors of EP-A-0324202 and, indeed the document does not suggest any quenchers which could be used in combination with the compounds it describes.

Acridinium compounds have been described (EP-A-0682254) which are capable of undergoing chemiluminescent reactions at pH values lower than 12-14 but these compounds are used as enzyme substrates and not labelling compounds for linking to ligands. EP-A-0682254 teaches that suitable molecules can be prepared using the methods set out in documents such as EP-A-0324202 but specifically states that reactive groups required for the covalent coupling to substances of biological interest are not a feature of the compounds alluded to therein. Therefore, these previously described compounds are structurally completely distinct from the compounds of the present invention and they would not be suitable for use as labelling compounds. It is not possible to predict the effect on the pH for chemiluminescent initiation of adapting the molecules of EP-A-0682254 to include a coupling group and the coupling of such an adapted molecule to a substance of biological interest.

A further problem with chemiluminescence energy transfer reactions is that, in addition to the chemiluminescent labelling molecule, they require a suitable quencher which has an absorption spectrum which overlaps at least partially, but preferably completely, with the emission spectrum of the labelling molecule. This might not seem to be a problem given that large numbers of quenchers are known but, in practice, it is no simple matter because the spectral range of both the labelling molecule and the quencher may vary considerably with the pH of their environment.

Therefore, a chemiluminescent transfer system requires a chemiluminescent labelling molecule which emits chemiluminescent radiation at a pH which is sufficiently low to avoid dissociation of a binding complex and which also emits chemiluminescent radiation at that pH having a spectrum which overlaps with that of a suitable quencher.

Thus there is a need for molecules which can be used to combine the exquisite sensitivity of detection of chemiluminescent molecules with the convenience of resonance energy transfer ligand binding assays.

In a first aspect of the present invention there is provided a compound of general formula (I) 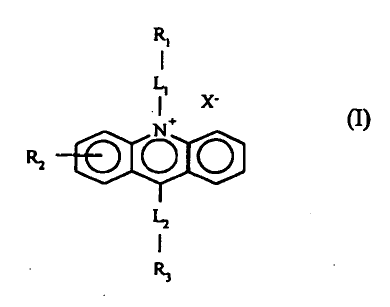 wherein:
either:
R₁ is a reactive group capable of reacting with an amine or thiol moiety;
L₁ is a hydrocarbon linker moiety comprising 2 - 12 carbon atoms, optionally substituted with hydroxy, halo, nitro or C₁-C₄ alkoxy; and
R₂ is hydrogen, C₁-C₄ alkyl, C₁-C₄ haloalkyl, aryl, fused aryl, C₁-C₄ alkoxy, C₁-C₄ acyl, halide, hydroxy or nitro;
or, alternatively:
the combination R₁-L₁- comprises a C₁₋C₄ alkyl group optionally substituted with hydroxy, halo, nitro or C₁-C₄ alkoxy; and
R₂ comprises a group R₄-L₁-, where R₄ is a reactive group capable of reacting
with an amine or thiol moiety; and L₁ is as defined above.
L₂ is -C(=O)O-, -C(=O)-S- or -C(=O)N(SO₂R₅)-,
wherein, in each case, the -C(=O) is linked to the ring carbon atom, and
R₅ is C₁-C₈ alkyl, aryl, C₁-C₈ alkoxy or C₁-C₈ acyl;
R₃ is a phenyl group substituted independently at the 2 and 6 positions with nitro, fluorine, chlorine, bromine or trifluoromethyl groups, wherein at least one of the said substituents is electron-withdrawing such that the pKa of the conjugate acid of the leaving group formed from R₃ and the -0, -S or -N(SO₂R₅) of the L₂ group is ≤ about 9.5; and
X⁻ is an anion formed as the result of the synthesis and processing of the molecule; wherein the compound may contain one or more additional R₂ moieties on either or both outer rings, provided that only one of said R₂ moieties may comprise an R₄-L₁- group;
thereby allowing production of chemiluminescent radiation on treatment with hydrogen peroxide in conditions maintained at about pH 6 to 10.

The substituents on the R₃ group are chosen such that the pKa of the conjugate acid of the leaving group formed by R₃ and the -0, -S or -N(SO₂R₅) of the L₂ group is ≤ about 9.5, which in practice means that at least one of the substituents on R₃ is electron withdrawing. This is a particularly important feature as it renders the molecule significantly chemiluminescent at pH 8 or less. This provides the compounds of the invention with an important advantage over the prior art compounds and, in particular the compounds exemplified in EP-A-0324202. The only ester compound exemplified in EP-A-0324202 (Examples 21 and 25) does not yield significant chemiluminescent emission at pH8 and, indeed requires a pH of at least 12 for useable chemiluminescent emission.

In contrast to prior art compounds, the chemiluminescence emission of the optimised acridinium labels of the present invention can be initiated at pH values compatible with commonly used quenchers and compatible with the stability requirements of ligand-binding complexes.

Thus, although EP-A-0324202 purports to relate to compounds which are useful as chemiluminescent labels, the problems outlined above are not identified. Furthermore, there is no example of the use of any compound in an energy-transfer assay and, indeed the general formula set out in the document encompasses numerous compounds which would be completely unusable in such an assay. The document also contains no teaching as to which quenchers might be used in combination with the compounds. The only example of a chemiluminescent acridinium ester in EP-A-0324202 contains a phenoxy substituent at the position equivalent to R₃ of the present invention and is reacted at a pH in excess of 12 for chemiluminescent emission, at which pH any binding complex would dissociate and many quenchers would be ineffective.

EP-A-0324202 describes intermediate ester compounds in Examples 1 to 8. Thus, numerous compounds are encompassed by EP-A-0324202 and yet the authors of that document show no appreciation of the importance of the structure of the compound for its purpose in energy transfer assays. Unsubstituted compounds, electron withdrawing compounds and electron donating compounds are given equal weight when, in fact, the present inventors have found that only electron withdrawing compounds are able to be used in energy transfer assays. The only measurement conditions described in EP-A-0324202 are pH ≥12, which results in dissociation of ligand binding complexes making energy transfer impossible. There is no teaching, or even appreciation, in the document as to which type of substituent is workable and the only acridinium ester precursor to be converted to a chemiluminescent final product is the compound of Example 1, which is an unsubstituted phenyl ester. Example 25 demonstrates that this compound is chemiluminescent when treated with basic hydrogen peroxide but there is no example of the linkage of a chemiluminescent compound to a ligand or of a chemiluminescence energy transfer assay. Indeed, as already mentioned, the particular compound exemplified could not be used in this way. It is therefore completely impossible to ascertain from this prior art document whether a particular compound would be useful in a chemiluminescence energy transfer assay. In some embodiments of the present invention, however, compounds in which R₃ is 4-chlorophenyl, 4-methoxyphenyl, 4-acetylphenyl or 1,1,1,3,3,3-hexafluoro-2-propyl may, in some circumstances be excluded from the definition of general formula (I).

In the context of the present invention, the term "ligand" refers to any moiety which is capable of binding specifically to a second moiety, referred to herein as an "anti-ligand" to form a complex. Examples of ligands and anti-ligands include antibodies and antigens or antigenic determinants; nucleic acid sequences; enzymes and enzyme substrates; and receptors and receptor-binding molecules.

"Labelling molecule" refers to a molecule which can be chemically bound to a ligand or anti-ligand and which emits a detectable signal enabling the ligand or anti-ligand to be detected. The labelling molecules of the present invention are chemiluminescent labelling molecules, which are capable of emitting chemiluminescent radiation on initiation of a suitable chemical reaction.

"Quencher" refers to a molecule which can be chemically bound to a ligand or anti-ligand and has an absorption spectrum which overlaps wholly or partially with the chemiluminescent or fluorescent emission spectrum of a labelling molecule such that when the quencher is brought into close proximity (typically less than 10nm) with the labelling molecule, the chemiluminescent or fluorescent emission spectrum is modulated.

"C₁-C₈ alkyl" refers to a straight or branched saturated hydrocarbon chain having up to eight carbon atoms. Examples include methyl, ethyl, propyl, isobutyl, t-butyl, n-hexyl and n-octyl. The term "C₁-C₄ alkyl" has a similar meaning but has from one to four carbon atoms.

"C₂-C₈ alkenyl" refers to a straight or branched hydrocarbon chain having from 2 to 8 carbon atoms and containing at least one carbon-carbon double bond. Examples include ethenyl, 2-propenyl and 3-hexenyl.

"C₂-C₈ alkynyl" refers to a straight or branched hydrocarbon chain having from 2 to 8 carbon atoms and containing at least one carbon-carbon triple bond. Examples include ethynyl, 2-propynyl and 3-hexynyl.

"Haloalkyl" refers to an alkyl group having one or more substituents selected from the group consisting of chloro, fluro, bromo and iodo.

"Aryl" refers to a 5 to 10 membered single or fused aromatic ring system which may contain one or more heteroatoms, for example nitrogen, oxygen or sulphur. Examples include phenyl, pyridyl, furanyl and naphthalenyl.

"Halo" refers to fluoro, chloro, bromo or iodo and "halide" to fluoride, chloride, bromide and iodide.

It will be appreciated that the means of coupling labelling molecules to biologically important molecules are well-established and that there are many variations of R₁ and L₁ which will permit the present invention to be practised. However, it has been found that the favourable results are achieved when L₁ comprises 2 to 10 carbon atoms. More preferably, L₁ is a fully saturated chain consisting of methylene units.

R₁ and R₄ groups which have been found to be particularly useful in linking the compound to a biologically important molecule include active esters, such as succinimidyl esters and imidate esters, maleimides and active halides such as chlorocarbonyl, bromocarbonyl, iodocarbonyl, chlorosulphonyl and fluorodinitrophenyl.

Similarly, it is well-established that there are numerous substitutions that could be represented by R₂ which allow the chemiluminescent activity to be retained and which can be used to alter the emission wavelength of the chemiluminescent label. This affects the choice of quencher used in an assay. The present inventors have found, however, that the compounds which are most useful include those in which R₂ is hydrogen or C₁-C₄ alkyl, especially methyl or ethyl as these are suitable for use in combination with the acceptor methyl red. Compounds in which R₂ is hydrogen are particularly preferred.

In preferred compounds of the present invention, L₂ is -C(=O)O-.

As already mentioned above, R₃ must be chosen such that the pKa of the conjugate acid of the leaving group formed by R₃ and the -0, -S or -N(SO₂R₅) is 5 about 9.5 and this means that it contains at least one electron-withdrawing group.

However, if the R₃ moiety forms a leaving group which is too reactive, this will mean that the compound is not always sufficiently stable to be useful as a labelling compound. For optimum efficiency as a labelling compound for chemiluminescent transfer assays, it is therefore preferred that the pKa of the conjugate acid of the leaving group formed by R₃ and the -0, -S or -N(SO₂R₅) of the L₂ group is >_ 3.

Therefore, in addition to electron withdrawing groups, the R₃ substituent caw contain groups that are not electron withdrawing and may be even electron donating provided that the effect of the electron withdrawing group that is present predominates. The structures of R₃ are readily predictable by calculation of the pKa values of the leaving groups resulting from the chemiluminescent reaction and therefore the scope of such groups can be appreciated by those skilled in the art

Examples of useful R₃ groups include 2,6-dibromophenyl, 2,6-bis(trifluoromethyl) phenyl and 2,6-diaiUophcnyl.

X- may be any one of a number of suitable anions; however, halide or halide-containing anions such as iodide, fluorosulfate, trifluoromethanesulfonate or tri fluoroacetate are preferred.

Compounds where R₂ is hydrogen or lower alkyl are particularly appropriate for use with methyl red as the energy acceptor as these compounds have the particular advantage that their spectrum of chemiluminescence emission lies completely within the absorption spectrum of the quencher methyl red at pH 9. This means that a chemiluminescence energy transfer system in which one of the preferred compounds set out above is used as the emitter and methyl red is the acceptor can be used to make quantitative measurements of the extent and rate of a reaction between a ligand and an anti-ligand and therefore the presence of substances or events that affect the said reaction. This may be done simply by measuring the extent of the change in the chemiluminescent signal.

As already mentioned, it is no simple matter to select a quencher which is suitable for use with any particular chemiluminescent emitter because the spectral range of both emitter and quencher may vary with the pH of their environment and the spectral overlap of these compounds of formula (I) with methyl red is particularly desirable.

Particularly preferred compounds of the invention include:
9-(2,6-bis(trifluoromethyl)phenoxycarbonyl)-10-(10-succinimidyloxycarbonyl ' decyl)acridinium trifluoromethanesulfonate;
9-(2,6-dibromophenoxycarbonyl)-10-(10-succinimidyloxycarbonyldecyl)a,cridinium trifluoromethanesulfonate;
9-(2,6-bis(trifluoromethyl)phenoxycarbonyl)-10-(3-succinimidyloxycarbonyl propyl)acridinium trifluoromethanesulfonate; and
9-(2,6-dibromophenoxycarbonyl)-10-(3-succinimidyloxycarbonylpropyl)acridinium trifluoromethanesulfonate;
9-(2,6-dinitrophenoxycarbonyl)-10-(10-succinimidyloxycarbonyldecyl)acridinium trifluoromethanesulfonate; and
9-(2,6-dinitrophenoxycarbonyl)-10-(3-succinimidyloxycarbonylpropyl)acridinium trifluoromethanesulfonate.

Compounds of the present invention may be prepared by well known methods. For example a compound of general formula (I) may be prepared by treating a compound of general formula (II): 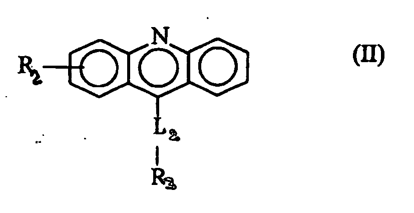
wherein R₂, L₂ and R₃ are as defined in general formula (I) with a compound of general formula (III)

   X-L₁-R₁ (III)
wherein L₁, R₁ and X are as defined in general formula (I), with X preferably being a leaving group such as triflate or iodide.

Typically, the reaction will be carried out in a polar organic solvent such as nitrobenzene and at elevated temperature of about 80 to 150°C.

Compounds of general formula (III) are well known and are readily available or can be prepared from readily available materials by known methods. For example, when the R₁ moiety of general formula (III) is a succinimidyl ester, it can be prepared by the reaction of N-hydroxysuccinimide with the corresponding carboxylic acid. '

Compounds of general formula (II) can be prepared by reacting a compound of general formula (TV) 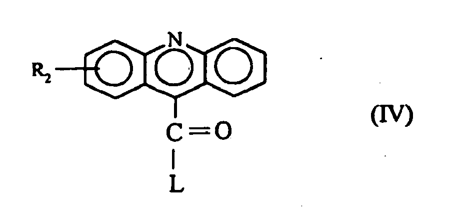
wherein R₂ is as defined in general formula (I) and L is a leaving group;
with a compound of general formula (V):

   H-Z-R₃ (V)
wherein R₃ is as defined in general formula (I) and Z is O or S.

The reaction may be conducted in a polar organic solvent such as pyridine and preferably at a temperature of from 10 to 50°C, but typically at room temperature.

Compounds of general formula (IV) are well known in the art and can be prepared by standard methods from compounds which are readily available. For example, acridine-9-carbonyl chloride can be prepared from acridine-9-carboxylic acid. Compounds of formula (V) are also readily available.

As discussed above, compounds of formula (I) emit chemiluminescence at a much lower pH than the compounds known in the prior art and this makes them particularly useful in a number of analytical methods.

Compounds of general formula (I) are particularly useful in any analytical method which uses chemiluminescence quenching to monitor association or dissociation of molecular species. For example, the cleavage of an enzyme substrate can be monitored by incorporating a compound of general formula (I) and a quencher respectively at either side of the point of cleavage such that the presence of active enzyme is determined by loss of quenching. Such methods are useful for identifying inhibitors or promoters of a given enzyme.

Similarly, chemiluminescent emitter/quencher pairs incorporating compounds of general formula (1) are useful in studying non-covalent binding processes such as are exemplified by immunoassay, protein binding, receptor binding, nucleic acid hybridisation and the like. Accordingly, such methods can be used to identify and/or quantify substances which affect such processes or are part of such processes. The bases of such determinations are well established and can be used together with the teaching herein disclosed to provide improved assay methods with the knowledge that one binding partner is labelled with a compound of general formula (I) and a further respective binding partner is labelled with a quencher or is itself a quencher. In certain circumstances, a binding partner may incorporate both emitter and quencher as exemplified below.

Therefore, in a further aspect of the invention there is provided a method of detecting or monitoring the association or dissociation of a first and a second binding partner in a medium, wherein the first binding partner is labelled with a compound of general formula (I) and the second binding partner is labelled with a quencher or is itself a quencher, such that when the first and second binding partners are associated, the chemiluminescent signal of the compound of general formula (I) is modulated; the method comprising adding hydrogen peroxide to the medium while maintaining the pH at about 6-10 and detecting the emission of chemiluminescent radiation.

The first and second binding partners may be molecular species which associate or dissociate *via* a covalent reaction which may be catalysed, for example by an enzyme. Alternatively, the first and second binding partners may be a ligand and an anti-ligand, for example complementary nucleic acids, an antibody and an antigen or antigenic determinant or a receptor and receptor binding molecule.

The method described above may also be adapted for the detection of an analyte in a medium. The analyte and the binding partners may be molecular species which associate or dissociate *via* a covalent reaction which may be catalysed, for example by an enzyme. Alternatively, they may be ligands and anti-ligands, for example complementary nucleic acids, an antibody and an antigen or antigenic determinant or a receptor and receptor binding molecule.

There is also provided a method of detecting the presence or absence in a medium of an analyte comprising a quencher for a compound of general formula (I), the method comprising:
a) adding to the medium a specific binding partner for the analyte labelled with a compound of general formula (I) such that if analyte is present a specific binding complex is formed;
b) adding hydrogen peroxide to the medium while maintaining the pH at about 6-10; and
c) detecting the emission of chemiluminescent radiation, wherein a modulated signal of the compound of general formula (I) indicates the presence of the analyte.

When the analyte is not a quencher for a compound of general formula (I), the invention provides a method for detecting an analyte in a medium, the method comprising:
a) adding to the medium a specific binding partner for the analyte labelled with a compound of general formula (I) or with a quencher for a compound of general formula (I) such that if analyte is present, a specific binding complex is formed;
b) adding to the medium simultaneously or sequentially a secondary binding reagent; and
c) detecting the presence of specific binding complex by a chemiluminescent method including the steps of adding hydrogen peroxide to the medium while maintaining the pH at about 6-10 and detecting the emission of chemiluminescent radiation, wherein a modulated signal of the compound of general formula (I) indicates the presence of the analyte.

In the method described above, the presence of analyte bound to the said first specific binding partner may be determined and/or quantified by either a competitive-binding assay or a "sandwich" assay. For the former assay, the secondary binding reagent is an analyte analogue labelled with either a quencher or a compound of general formula (I) as appropriate to the labelled first labelled specific binding partner. For the latter assay the secondary binding reagent is a second specific binding partner labelled with either a quencher or a compound of general formula (I) as appropriate to the first labelled specific binding partner. The general design of competitive and non-competitive binding assays is well-known to those skilled in the art and established principles can be applied to the present invention save that the specific teachings of the present invention are followed in regard to the specific needs of the improved compounds and associated chemiluminescence quenching system as disclosed herein.

In a further example of an assay of this aspect of the invention, the analyte is a nucleic acid and the assay employs an oligonucleotide probe as specific binding partner which is a single stranded nucleic acid comprising up to about 40 bases of which the two ends (about 4-8 bases) are mutually complementary and the central section contains a sequence of about 15 to 30 bases which is complementary to all or part of the target sequence of the analyte. The oligonucleotide probe may be labelled with a compound of general formula (I) at one end and a quencher at the other. In the absence of analyte, the mutually complementary ends of the oligonucleotide probe will bind to one another bringing the two ends of the molecule into contact such that any chemiluminescent signal emitted by the molecule of general formula (I) will be modulated by the quencher. In the presence of analyte, however, the central section of the molecule will bind to the analyte more strongly than the binding of the two ends of the molecule. Therefore, in the presence of analyte, the quencher will be separated from the compound of general formula (I) so that no quenching of the chemiluminescent radiation will occur.

In a further modification, the method can be adapted to detect the presence of inhibitors or promoters of a binding process. The method comprises mixing a first and a second specific binding partner labelled respectively with a compound of general formula (I) and a quencher, adding hydrogen peroxide to the medium while maintaining the pH at about 6-10 and detecting the emission of chemiluminescent radiation, wherein modulation of the chemiluminescent signal of the compound of general formula (I) indicates promotion of the binding process and the presence of an unmodulated signal indicates inhibition of the binding process.

As mentioned above, one example is the use of the method to monitor the cleavage of an enzyme substrate, in which case the first and second binding partners will be molecular species (peptides) covalently attached to each other, labelled at sites respectively either side of a cleavage site with a compound of general formula (I) and a quencher such that cleavage of the substrate may be determined by loss of quenching.

In a further example, each of two complementary strands of nucleic acid is labelled respectively with a compound of general formula (I) and a quencher. The relative labelling positions must be selected such that the emitter and quencher are in sufficiently close proximity to one another to interact when the complementary strands are bound in a duplex. Therefore, chemiluminescence is only observed when the strands are substantially separated, for example by a process such as enzyme activity, temperature change, competition for binding with unlabelled oligonucleotides, chaotropic agents and the like.

Preferred compounds of general formula (I) for use in this assay method are as described above for the first aspect.

When the compound of general formula (I) is one of these preferred compounds, it may be used in combination with methyl red as a quencher since the chemiluminescent emission spectrum of the preferred compounds falls entirely within the absorption spectrum of methyl red at pH 6-10.

The assay methods described above are more sensitive than the corresponding assay using fluorescent detection methods. Therefore, a further advantage of chemiluminescent detection methods is that they may obviate the need for steps such as nucleic acid amplification, which is often used in conjunction with fluorescent detection methods simply to increase the amount of analyte present sufficiently for it to fall within the detection limits of the assay.

The assay methods of the present invention are simple to carry out and are suitable for the detection of a wide range of analytes.

In a further aspect of the invention, there is provided a reagent comprising a ligand or anti-ligand labelled with a compound of general formula (I). The ligand or anti-ligand may be a labelled nucleic acid or a peptide or protein, for example an antibody, antigen, antigenic determinant, enzyme, enzyme substrate, receptor or receptor binding molecule. The reagent is suitable for use in the assay methods described above.

In some cases the ligand or anti-ligand may further be labelled with a quencher. For example, the reagent may comprise a nucleic acid molecule comprising up to about 40 bases wherein the ends are mutually complementary and the central section contains a sequence of about 15 to 30 bases which is complementary to a target sequence and wherein the molecule is labelled at one end with a compound of general formula (I) and at the other end with a quencher.

Alternatively, the ligand or anti-ligand may comprise a nucleic acid duplex each strand of which is labelled respectively with a compound of general formula (I) and a quencher such that attenuation of the light emission exists when the duplex is substantially intact but not when the duplex is rendered substantially non-intact by chemical or physical means.

A further example of such a reagent comprises an enzyme substrate labelled at sites respectively either side of a cleavage site with a compound of general formula (I) and a quencher such that cleavage of the substrate may be determined by loss of quenching.

Preferred compounds of general formula (I) are as described above and when one of these preferred compounds is used, the quencher may be methyl red.

The reagent may form part of a kit and therefore the invention further provides a kit comprising a reagent as described above, hydrogen peroxide solution and a buffer adapted to adjust the pH of the analyte containing medium to pH 6-10, preferably about pH 9.

As mentioned above, the reagent may comprise a quencher in addition to the compound of general formula (I), for example when the ligand or anti-ligand is a nucleic acid molecule or an enzyme substrate.

Alternatively, the reagent may comprise either a specific binding partner for an analyte or a secondary binding partner and the kit may further comprise a second reagent labelled with a quencher. Thus, if the reagent comprise a specific binding partner for an analyte, the second reagent will comprise a secondary binding partner, and *vice versa*.

When the kit is adapted for use in a competitive binding assay, the secondary binding reagent comprises an analyte analogue labelled with a quencher or a compound of general formula (I) as appropriate to the labelled first labelled specific binding partner. For sandwich assays, the secondary binding reagent comprises a second specific binding partner labelled with either a quencher or a compound of general formula (I) as appropriate to the first labelled specific binding partner.

The reagent and the second reagent may be provided either as a mixture or as separate components of the kit.

The invention will now be described in greater detail with reference to the following non-limiting examples.

In a plot which shows the absorption spectra of the quenchers Dabcyl and methyl red at pH 9 plotted on the same axes as the emission spectrum of acridinium ester, at this pH, the emission spectrum of the acridinium ester overlaps completely with that of methyl red.

In a plot of amount of target sequence in a test solution against chemiluminescence intensity obtained from a nucleic acid probe having complementary end sequences labelled respectively with a compound of general formula (I) and methyl red, as the amount of target sequence in the solution increases, the hybridisation of the two end sequences of the probe to one another decreases and therefore the amount of quenching of the chemiluminescent signal also decreases.

In the following examples, the compounds which are synthesised and tested are all compounds in which R₁ is a reactive group capable of reacting with an amine or thiol moiety and L₁ is a linker. However, compounds which can be linked to a ligand *via* the R₂ moiety (i.e. compounds in which R₂ is R₄-L₁) are also useful in the present invention. The principle of linking chemiluminescent compounds of this type to a ligand *via* an outer ring substitutent (R₂ in general formula (I)) is well known in the prior art, for example from US 5283334 (McCapra), and it has been demonstrated that such compounds are chemiluminescent.

### Example 1 - Preparation of 9-(2,6-dibromophenoxycarbonyl)-10-(10-succinimidyloxy carbonyldecyl)acridinium trifluoromethanesulfonate

### a. 11-iodoundecanoic acid^{[8, 9]}

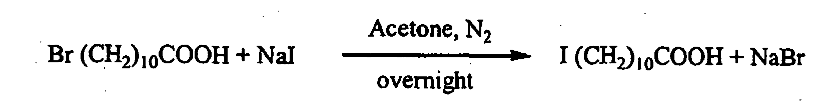

To a 50 ml flask, 11-bromoundecanoic acid (2.192 g, 8.27 mmol) and NaI (3.0370 g, 20.3 mmol) were added and the mixture was dried over an oil pump for 1 h. Acetone (20 ml) was added and the mixture was stirred for 20 h. The resulting mixture was poured into water (20 ml) and extracted with diethyl ether (25 ml × 4), then the combined organic extracts were washed with saturated NaSO₃, dried with MgSO₄, filtered and evaporated to give a crude pale red solid product (2.3609 g, 91.5% yield). The solid was purified by column chromatography (silica, hexane + ether +formic acid, 100:100:0.1). The fractions of Rf = 0.49 were collected and evaporated to dryness to yield a white solid (2.230g, 83%, m. p, 63-64 °C^{[8]}. ¹HNMR, (CDCl₃, δ, ppm), 3.12 (2H, t, J = 7 Hz), 2.28 (2H, t, J = 7 Hz), 1.75 (2H, quintet, J = 7 Hz), 1.56 (2H, quintet, J = 7 Hz), 1.24-1.18 (12H, m); ¹³CNMR (CDCl₃, δ, ppm), 179.9 (C=O), 34.3, 33.9, 30.8, 29.7, 29.6, 29.5, 29.4, 28.9, 25.0, 7.5 (10 x CH₂); IR (υ, cm⁻¹) 1693 (C=O); MS (EI) 313 (M + H⁺, 3%), (CI) 330 (M + NH₄⁺, 100%).

### b. Succinimidyl 11-iodoundecanoate^{[10]}

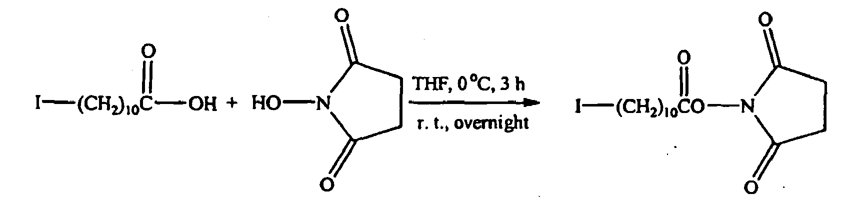

To a 50ml flask, 11-iodoundecanoic acid (556 mg, 1.78 mmol), N-hydroxysuccinimide (440 mg, 3.83 mmol) and dry THF (10 ml) were added and the system was flushed with nitrogen. The mixture was cooled in an ice bath for 10 min. then dicyclohexylcarbodiimide (DCC, 770 mg, 3.76 mmol) in THF (10 ml) was introduced. The mixture was maintained at 0 °C for 3 h, then at room temperature overnight. The resulting mixture was filtered to remove most of the side product dicyclohexylurea (DCU), the filtrate was concentrated under reduced pressure and the residue was then loaded onto a silica gel column for chromatography. The column was eluted with toluene + ethyl acetate (4:1) and the fractions with Rf = 0.59 were combined and evaporated to give a white solid (639 mg, mp 83 °C). The impurities were removed by crystallization from hexane + EtOAc at 65 °C, to yield the pure product (387 mg, 53.%). ¹HNMR, (CDCl₃, δ, ppm), 3.16 (2H, t, J = 7 Hz), 2.81 (4H, s), 2.57 (2H, t, J = 7 Hz), 1.79, (2H, quintet, J = 7 Hz), 1.71 (2H, quintet, J = 7 Hz), 1.41-1.22 (12H, m); ¹³CNMR (CDCl₃, δ, ppm) 169.6, 169.1 (2 x C=O), 34.0, 31.3, 30.9, 29.7, 29.6, 29.4, 29.1, 28.9, 26.0, 25.0, 7.7 (12 x CH₂); IR (υ, cm⁻¹) 1725; MS (CI) 427 (M+NH₄⁺, 15%), 301 (15%), 225 (16%), 149 (30%), 132 (100%).

### c. Succinimidyl 11-(trifluoromethanesulfonyloxy)undecanoate^{[11-13]}

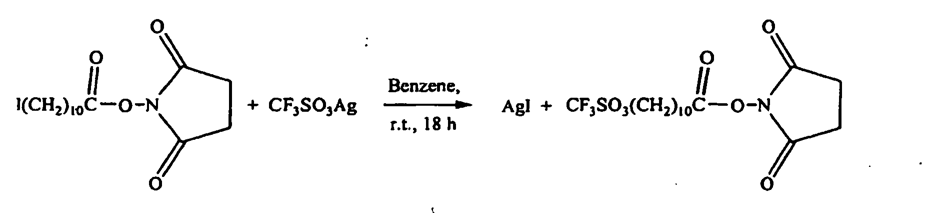

To a 5 ml flask, succinimidyl 11-undecanoate (188 mg, 0.460 mmol) and silver triflate (214 mg, 0.836mmol) were added, and the flask was flushed with N₂ for 5 min. Benzene (1.5ml) was introduced with a syringe, whereupon some yellow solid appeared immediately. The mixture was stirred for 18 h at room temperature, then was loaded onto a short chromatography column (silica gel, 2g), which was eluted with dichloromethane (DCM). The fractions with Rf = 0.48 were combined and evaporated to give a white gel (133 mg, yield, 67%).
¹HNMR (CDCl₃, δ, ppm), 4.44 (2H, t, J = 6 Hz) 2.75 (4H, s), 2.51 (2H t, J = 7 Hz), 1.73 (2H, m), 1.63 (2H, quintet, J = 7 Hz), 1.37-1.17, (12H, m); ¹⁹FNMR (CDCl₃, δ, ppm), -74.8; ¹³CNMR (CDCl₃, δ, ppm), 169.6, 169.0 (C=O), 119.0 (quartet, CF₃SO₃), 78.1, 31.3, 29.6, 29.5, 29.4, 29.3, 29.2, 29.1, 26.0, 25.4, 24.9 (12 x CH₂); IR (υ, cm⁻¹), 1725.

### d. 2,6-dibromophenyl acridine-9-carboxylate^{[3-5]}

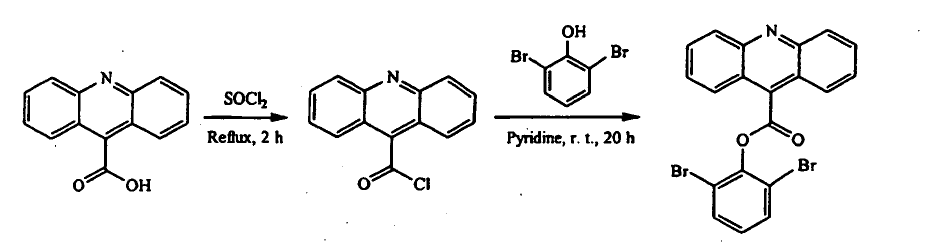

In a round bottom flask (50 ml) equipped with a CaCl₂ drying tube and a magnetic stirrer bar, acridinium-9-carboxylic acid chloride (2.036g, 8.42 mmol) in pyridine was heated to (70 °C). After the solid had dissolved completely, the solution was cooled to room temperature, 2,6-dibromophenol (2.325 g, 9.23 mmol) was added and the mixture was stirred vigorously overnight. The resulting mixture was stripped of pyridine to leave a brown solid. DCM (10 ml) was used to extract the product and the extract was subjected to column chromatography, eluted with toluene + EtOAc (4: 1). The fractions with Rf 0.51 were collected and evaporated to give a pale yellow solid. The solid was re-dissolved in DCM (40 ml), washed with dilute NaOH (5 x 40 ml), dried over MgSO₄, filtered and evaporated to give a pale yellow powder (2.080 g, 54% yield), mp 159-160 °C.

### e. 9-(2,6-dibromophenoxycarbonyl)-10-(10-succinimidyloxycarbonyl decyl)acridinium trifluoromethanesulfonate^{[6, 7]}

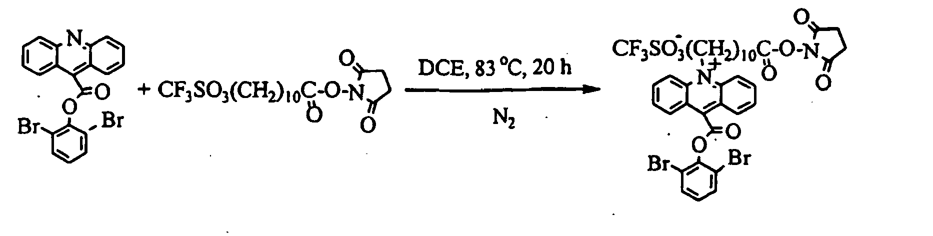

To a 5 ml flask, 2,6-dibromophenyl acridine-9-carboxylate (93.2 mg, 0.204 mmol), succinimidyl 11-(trifluoromethanesulfonyloxy)undecanoate (94.1 mg, 0.218 mmol), and a stirrer bar were added, then the flask was equipped with an air condenser and flushed with N₂. Tetrachloroethane (1.5 ml) was introduced by a syringe, whereupon the mixture turned to bright yellow. The flask was placed into an oil bath (140 °C) for 4 h, during which the mixture gradually turned to orange and eventually a brown-red solution was obtained. The whole mixture was stripped of solvents and then extracted with DCM and precipitated with EtOEt. The precipitate was re-dissolved in DCM and precipitated repeatedly until TLC showed no presence of starting materials. A yellow solid (28 mg, 15% yield, mp 165-166 °C) was obtained and characterized. ¹HNMR, (CDCl₃, δ, ppm), 8.84 (2H, d, J = 8 Hz), 8.66 (2H, d, J = 9 Hz), 8.39 (2H, dd, J = 8, 7 Hz), 7.89 (2H, dd, J = 9, 7 Hz), 7.58 (2H, d, J = 8 Hz), 7.06 (1H, t, J = 8 Hz), 5.47 (2H, t, J = 8 Hz), 2.65 (4H, s), 2.41 (2H, t, J = 7 Hz), 2.03 (2H, m), 1.64-1.49 (4H, m), 1.31-1.07 (10H, m); ¹⁹FNMR (CDCl₃, δ, ppm), -78.2; ¹³CNMR (CDCl₃, δ, ppm), 169.7, 169.1, 161.5 (3 x C=O), 147.1, 145.8, 141.7, 140.8, 133.8, 130.3, 129.9, 129.3, 123.9, 119.8, 117.7, 52.9, 31.3, 30.1, 29.6, 29.5, 29.4, 29.3, 29.0, 26.9, 26.0, 24.9; IR (υ, cm⁻¹) 1774, 1728 (C=O); MS (ES-), 149 (100%, CF₃SO₃); (ES+), 739 (80%, M-CF₃SO₃), 519 (100%).

### Example 2 - Preparation of 9-(2,6-dibromophenoxycarbonyl)-10-(3-succinimidyloxy carbonylpropyl)acridinium trifluoromethanesulfonate

### a. Succinimidyl 4-Iodobutanoate

4-Iodobutyric acid (115 mg, 0.54 mmol) in tetrahydrofuran (THF, 5 ml) was cooled to 0 °C. To the solution, N-hydroxysuccinimide (65 mg, 0.57 mmol) in THF (1 ml) and dicyclohexylcarbodiimide (125 mg, 0.61 mmol) in THF (2 ml) were added respectively. The mixture was stirred at 0 °C for 3 h and then at room temperature overnight. The suspension obtained was filtered. The filtrate was concentrated and purified by silica gel chromatography (Toluene/ethyl acetate (Tol-EtOAc), 4: 1). The desired product (132 mg) was obtained as a pale yellow solid, mp 86-87°C.

### b. 9-(2,6-Dibromophenoxycarbonyl)-10-(3-succinimidyloxy carbonyl)propyl)acridinium iodide

2,6-Dibromophenyl acridine-9-carboxylate synthesised as in Example 1d (43 mg, 0.094 mmol) and succinimidyl 4-iodobutanoate (50 mg, 0.16 mmol) in nitrobenzene (2 ml) in a glass tube were heated at 120 °C on an oil bath for 2 hours. The brown solution obtained was washed with petroleum ether (40-60) (5 x 2 ml) to give a brown solid (about 2 mg). The washings were combined and evaporated on a rotary evaporator and then under vacuum for 2h to get an orange solution. The mixture was reused. The experiment was carried out under the conditions described in Example 1e.

### Example 3 - Preparation of 9-(2,6-bis(trifluoromethyl)phenoxycarbonyl)-10-(10-succinimidyloxycarbonyldecyl)acridinium trifluoromethanesulfonate

### a. 2-trifluoromethyl-6-iodophenol^{[1, 2]}

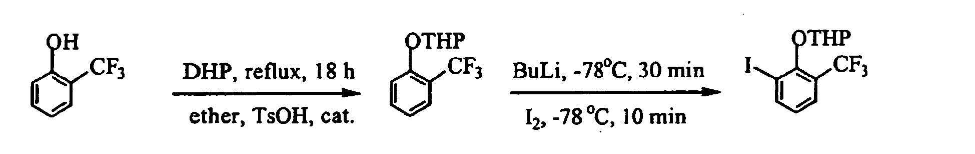

To a 50 ml flask, 2-trifluoromethylphenol (1.00 g, 6.17 mmol) and 3,4-dihydropyran (2 ml) were added, and then a few crystals of toluenesulfonic acid monohydrate (TsOH.H₂O) were added. After ether (25 ml) was introduced, the flask was placed in an oil bath and refluxed for 19 h. The resulting mixture was poured into saturated NaHCO₃ (20 ml), the aqueous layer was extracted with diethyl ether (25 ml × 2), and the combined extracts were washed with saturated NaCl, dried over MgSO₄ and stripped of solvents to give a colourless oil, 2-(trifluoromethyl)phenol tetrahydropyranyl ether (1.35 g, 5.50 mmol, 89% yield). ¹HNMR (CDCl₃, δ, ppm), 7.50 (1H, d, J = 8Hz), 7.38 (1H, t, J = 8Hz), 7.18 (1H, d, J = 8Hz), 6.94 (1H, t, J = 8 Hz), 5.48 (1H, approx. t, J = 3 Hz), 3.77 (1H, dt, J= 3, 11 Hz), 3.55 (1H, m), 1.96 (1H, m), 1.89-1.73 (2H, m), 1.71-1.46 (3H, m). The oil was dissolved in THF (8.5 ml) and treated with BuLi (3 ml of 2.5 M, 7.5 mmol); after stirring at -78 °C for 30mins, the reaction mixture was treated with a solution of iodine (2.2013 g, 8.67 mmol) in THF (8.5 ml), stirred for 10 min, and then allowed to warm to room temperature. The mixture was poured into 20% Na₂SO₃ and vigorously shaken. The aqueous layer was extracted with diethyl ether and the combined extract was washed with saturated NaCl and then dried over MgSO₄. After stripping of solvent, a yellow-red oil was obtained. ¹HNMR (CDCl₃, δ, ppm), 7.88 (1H, dd, J = 8, 1 Hz), 7.46 (1H, dd, J = 8, 1 Hz), 6.80 (1H, t, J = 8Hz), 5.25 (1H, dd, J = 5, 2 Hz), 4.04 (1H, m), 3.43 (1H, m), 2.01 (1H, m), 1.91-1.79 (2H, m), 1.58-1.42 (3H, m). This showed the oil to be 2-(trifluoromethyl)-6-iodophenol tetrahydropyranyl ether. The oil was subjected to short-path distillation, during which decomposition of the tetrahydropyranyl ether took place, giving the product as 2-(trifluoromethyl)-6-iodophenol (1.667 g, yield, 94% yield if assumed to be pure, but in reality contaminated by about 15% of dihydropyran hydrate); ¹HNMR (CDCl₃, δ, ppm), 7.88 (1H, d, J = 8 Hz), 7.47 (1H, d, J = 8 Hz), 6.79 (1H, t, J = 8 Hz), 5.85 (1H, s).

### b. 2,6-bis(trifluoromethyl)phenol^{[1, 2]}

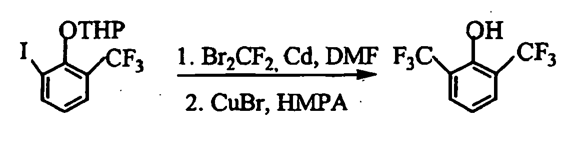

To a 50 ml flask equipped with a stirrer bar, Cd powder (3.057 g, 27.2 mmol) and dimethyl formamide (DMF, 10 ml) were added. The flask was flushed with N₂ and then cooled to 0 °C in an ice bath. CBr₂F₂ (1ml + 1ml + 0.5ml, 27.17 mmol in total) was added slowly by syringe, then the mixture was allowed to warm up to room temperature and stirred for an additional 30min. The mixture was diluted with hexamethylphosphoramide (HMPA, 10 ml) and cooled to 0 °C, CuBr (1.8036 g, 12.57 mmol) was added and the whole was stirred for 10min. To the resultant mixture containing F₃CCu was added 2-trifluoromethyl-6-iodophenol (937.1 mg, 3.25 mmol) and the mixture was heated over an oil bath (65 °C) with stirring for 2h. The whole mixture was poured into a mixture of 3M HCl (30 ml) and ether (30 ml), the solids were removed from the mixture by filtration and the two liquid layers were separated. The aqueous phase was extracted with further ether (25 ml × 5), and the combined extract was washed with saturated NaCl and then dried over MgSO₄. The solvent was removed by rotary evaporation and the residue was subjected to short path distillation. A fraction with bp 70 °C (1 mbar) was collected; yield 283.4 mg, 38%. ¹HNMR (CDCl₃, δ, ppm), 7.70 (2H, d, J = 8 Hz), 7.06 (1H, t, J = 8 Hz); ¹³CNMR (CDCl₃, δ, ppm), 153.0, 131.0, 123.8 (q due to coupling to 3 F nuclei), 120.3, 120.0. EI-MS, m/z, 230 (M⁺, 18%), 213 (28%), 179 (23%), 163 (30%), 135 (100%).

### c. 2,6-bis(trifluoromethyl)phenyl acridine-9-carboxylate^{[3-5]}

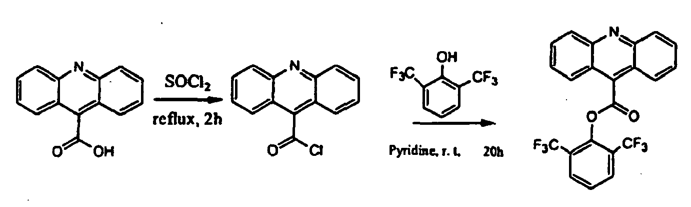

In a 5ml flask equipped with a stirrer bar, a condenser, and a CaCl₂ drying tube, acridine-9-carboxylic acid chloride (131.6 mg, 0.544 mmol), 2,6-bis(trifluoromethyl)phenol (130 mg, 0.565 mmol) and pyridine (3 ml) were mixed and stirred vigorously at 40 °C for 1 day. The solvent was evaporated from the mixture, and the residual solid was subjected to column chromatography (silica, toluene:EtOAc 4:1). The fractions with Rf = 0.49 were collected and evaporated to give an orange solid (137.5 mg, 58% yield), mp 171 °C; ¹HNMR (CDCl₃, δ, ppm), 8.51 (2H, d, J = 9 Hz), 8.32 (2H, d, J = 9 Hz), 7.96 (2H, d, J = 8 Hz), 7.79 (2H, dd, J = 9. 7 Hz), 7.61 (2H, dd, J = 9, 7 Hz), 7.56 (1H, t, J = 8 Hz); ¹³CNMR (CDCl₃, δ, ppm), 164.6 (C=O), 148.6, 146.3, 131.9, 131.0, 130.0, 128.5, 127.8, 126.5, 126.2, 125.8, 123.8, 122.8 (q due to coupling to 3 F nuclei); IR (υ, cm⁻¹), 1758 (C=O); MS: (EI), m/z, 435 (M⁺, 10%), 206 (82%), 178 (100%); (CI), m/z, 436 (M+H⁺, 39%), 180 (100%).

### d. 9-(2,6-bis(trifluoromethyl)phenoxycarbonyl)-10-(10-succinimidyl oxycarbonyldecyl)acridinium trifluoromethanesulfonate^{[6, 7]}

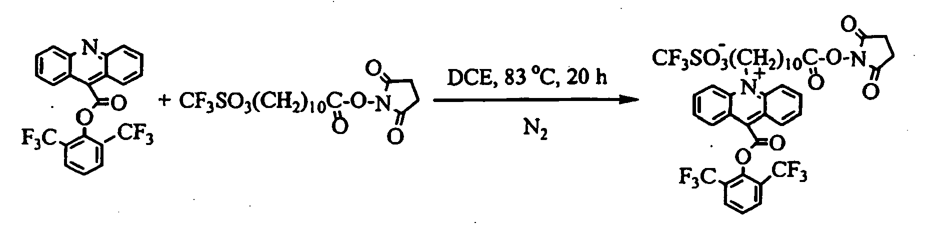

To a 5 ml flask previously flushed with nitrogen, 2,6-bis(trifluoromethyl)phenyl acridinium-9-carboxylate (74.9 mg, 0.172 mmol) and succinimidyl 11-(trifluoromethanesulfonyloxy)undecanoate (prepared according to Example 1c, 74.8 mg, 0.174 mmol) were added. The flask was re-flushed with N₂, then dry dichloroethane (1.6 ml) was introduced by syringe. The mixture was maintained at 80 °C for 20 h and afterwards stripped of solvents on a rotary evaporator. The mixture was extracted with dichloromethane (1 ml), then the extract was subjected to column chromatography on silica gel, eluted by dichloromethane (DCM)/CH₃CN (3: 1). The component with Rf = 0.19 was collected and evaporated to give a yellow solid (15.9 mg, 10.7% yield), mp 164-165 °C; ¹HNMR (CDCl₃, δ, ppm) 8.88 (2H, d, J = 9 Hz), 8.77 (2H, d, J = 9 Hz), 8.58 (2H, dd, J = 9, 7 Hz), 8.15 (2H, d, J = 8 Hz), 8.07 (2H, dd, 9, 7 Hz), 7.81, (1H, t, J = 8 Hz), 5.69 (2H, t, J = 8 Hz), 2.85 (4H, s), 2.60 (2H, t, J = 7 Hz), 2.29-2.18 (2H, m), 1.84-1.69 (4H, m), 1.51-1.26 (10H, m); ¹³CNMR (CDCl₃, δ, ppm), 169.7, 169.1, 161.8, 144.9, 141.7, 140.6, 132.4, 130.0, 129.0, 128.5, 126.0, 125.7, 124.3, 119.8, 53.3, 31.3, 30.1, 29.6, 29.5 (2 signals), 29.3, 29.0, 26.9, 26.0, 24.9 (the CF₃ signals were not seen as the signal to noise ratio was too low); MS (ES+) 717 (C₃₇H₃₅F₆N₂O₆⁺, 25%), (ES-) 149 (CF₃SO₃⁻, 100%).

Methods similar to those of Examples 1 to 3 have been used to prepare 9-(2,6-dinitrophenoxycarbonyl)-10-(10-succinimidyloxycarbonyldecyl)acridinium trifluoromethanesulfonate and 9-(2,6-dinitrophenoxycarbonyl)-10-(3-succinimidyl oxycarbonylpropyl)acridiniumtrifluoromethanesulfonate.

### Example 4 - Effect of Variation of X Group on Chemiluminescence

This example shows that not all compounds having X groups as taught in EP-A-0324202 are capable of undergoing a chemiluminescent reaction at moderate pH. Compounds of the following general formula were synthesised according to established methods:

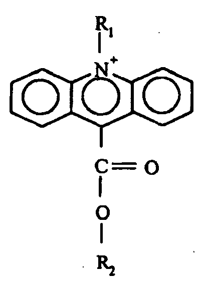

In the above formula:
In Compound (a), R₁ is methyl, R₂ is phenyl;
In Compound (b), R₁ is methyl, R₂ is 3-methylphenyl;
In Compound (c), R₁ is methyl, R₂ is 2,6-difluorophenyl;
In Compound (d), R₁ is (succinimidyloxycarbonyl)propyl, R₂ is 2,6-dibromophenyl

Chemiluminescence from these compounds was measured using a luminometer (Lumino^{™}, Stratec Electronik GmbH, Pfortzheim, Germany) following onboard initiation of the reaction by injection (200µl) of buffered hydrogen peroxide of pH 9 or pH 12. pH 9 was achieved using disodium hydrogen orthophosphate (0.02M/l), pH 12 was achieved as set forth in EP-A-0324202 by diluting 100µl of 5M aqueous sodium hydroxide and 20µl of 30% aqueous hydrogen peroxide in 20ml water. In both cases, 30% hydrogen peroxide was used.

The measurement time was 5 seconds, over which time photon counts were integrated and began immediately the reagent was injected. The amounts of each compound used and the luminometer counting factor were chosen so that each compound exhibited comparable peak heights at pH 12. Nominally, concentrations in the range 1-10ng/ml were used with the pure compounds being first dissolved in acetonitrile at a concentration of 1mg/ml and then further diluted in mM/l hydrochloric acid. 10µl volumes were used for luminometry.

**Table 1**

| | **Compound** | | | |
|---|---|---|---|---|
| | (a) | (b) | (c) | (d) |
| **pH 12** | 100 | 100 | 100 | 100 |
| **pH 9** | 7 | 3 | 100 | 100 |

Table 1 shows the chemiluminescent emission intensity at pH 9 as a percentage of that obtained using the conventional reaction conditions (pH 12) set out in EP-A-0324202. It can be seen that, although all four compounds possess X groups falling within the scope of EP-A-0324202, only compounds (c) and (d) are capable of emitting significant chemiluminescence at moderate pH. These two compounds have phenyl moieties which possess electron withdrawing groups as required by the present invention.

### Example 5 - Effect of pH on Stability of Binding Complexes

A stem loop oligonucleotide of the following sequence possessing respective end modifications comprising methyl red (MR) and a primary amine linker was synthesised to order and purchased commercially (Oswel Research Products, Romsey, UK).
NH₂-5'-CCGGTCCGGGCAATGACAGCAAAAACAGGGACCGGT-MR

The primary amine was labelled with the acridinium ester Compound (d) from Example 2 using succinimidyl ester chemistry by established methods. (Nelson et al, Nonisotopic Probing, Blotting and Sequencing , pp. 391-428, Academic Press, 1995) Clearly, neither Compound (a) nor Compound (b) could be used as the label because, as seen in Table 1 above, they do not emit significant chemiluminescence at pH 9. However, Compound (d) has the property of retaining 100% of its pH 12 emission intensity when the pH is reduced to 9.

The chemiluminescence emitted by the oligonucleotide labelled with Compound (d) was measured at pH 9 and pH 12 using the method set out in Example 2 and the results are shown in Table 2.

**Table 2**

| | Unquenched maximal RLU* (0% quenching) | Actual RLU observed | % quenching |
|---|---|---|---|
| pH 9 | 1153347 | 24832 (2% of maximum) | 98 |
| pH 12 | 1326487 | 1299306 (98% of maximum) | 2 |

| | | | |
|---|---|---|---|
| RLU = relative light units. | | | |

Table 2 shows that at pH 9, the chemiluminescent signal emitted by Compound (d) is 98% quenched by methyl red but at pH 12, the quenching is only 2%.

The oligonucleotide labelled with Compound (d) exists in free solution under mild conditions (pH 9) as a stem-loop structure due to the formation of a stable intramolecular binding complex of Structure (A)
(A) 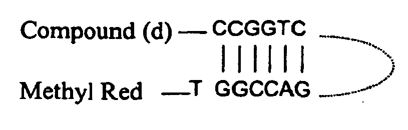
(Quenching present)

Thus, at pH 9, minimal background chemiluminescence emission is seen due to chemiluminescence quenching arising from the close proximity of the quencher, methyl red, to the emitter, Compound (d).

In contrast, when chemiluminescence is initiated at pH 12 as set out in EP-A-0324202, significant chemiluminescent emission is observed. This is because, at pH 12, hybridisation between the complementary nucleic acids at the 5' and 3' terminals of the oligonucleotide is disrupted and so the labelled oligonucleotide adopts structure (B).
(B) Compound (d) - CCGGTC -----GACCGG T - Methyl Red
(Quenching absent)

In structure (B), the emitter Compound (d) is not sufficiently close to the quencher, methyl red, for quenching to occur.

From the results of this experiment, it is clear that chemiluminescence quenching cannot be used as the basis of an assay system under the conditions taught in EP-A-0324202 since the binding complex is non-specifically dissociated under these conditions. The same is true for systems other than nucleic acid systems, for example those based on antibody-antigen binding.

By contrast, the binding complex remains intact at pH 9, permitting quenching to occur. Thus, only acridinium labels which undergo a chemiluminescent reaction at moderate pH such as those described herein can be used as the basis of a homogeneous energy transfer binding assay.

### Example 6 - Choice of a Quencher for use with Compounds of Formula (I)

EP-A-0324202 suggests energy transfer acceptors such as fluorescein and Lucifer Yellow which re-emit the absorbed energy at a different wavelength from that of the donor and thus such acceptors cannot be used as quenchers. No indication is given in the document of any other useful quenchers for the claimed compounds. However, fluorescence quenchers have been described and it might be expected that commonly used fluorescent quenchers could be employed. This example demonstrates that the well established quencher Dabcyl^{™} cannot, as might have been expected, be used to quench the emission from acridinium emitters under all circumstances. This is because the absorption spectrum of the quencher at the pH required to bring about the chemiluminescent reaction shifts relative to the emission spectrum of the acridinium label.

The emission spectrum of the primary emitter of the acridinium ester chemiluminescence (N-methylacridone) is well established. The absorption spectra of methyl red and Dabcyl^{™} were measured in a spectrofluorimeter. These spectra were overlaid with that of the emission spectrum of N-methylacridone to yield a composite spectrum. There is only a modest overlap between the emission spectrum of N-methylacridone and the absorption spectrum of Dabcyl^{™} at pH 9. Since efficient resonance energy transfer quenching requires maximal spectral overlap, it can be seen that such Dabcyl^{™} quenching is inefficient at the pH required to initiate the chemiluminescent reaction of the preferred acridinium esters. By contrast, the preferred compounds of the present invention exhibit 100% spectral overlap with the methyl red quencher at pH 9. Thus, only a methyl red quencher and not the conventionally used Dabcyl^{™} is an efficient quencher of acridinium esters which are capable of undergoing a chemiluminescent reaction at pH 9. This is somewhat unexpected since Dabcyl^{™} has been stated to be a useful quencher in many energy transfer assays.

### Example 7 - Chemiluminescent Properties of Compounds of Formula (I)

This example demonstrates that the compounds of the present invention can be used as the basis of a homogeneous energy transfer quenching binding assay since such compounds can exhibit chemiluminescence at moderate pH and are capable of acting as part of efficient quenching systems in conjunction with carefully selected quenchers, such as methyl red. The example also demonstrates that, by contrast, acridinium esters which require elevated pH to bring about chemiluminescence are incapable of being used for such purpose as is the case with numerous compounds which fall within the scope of EP-A-0324202.

The labelled stem loop oligonucleotide described in Example 3 (1-10fM) in 10µl hybridisation buffer (0.1M/l lithium succinate, pH 5.1; 8.5% w/v lithium lauryl sulphate; 0.125M/l lithium hydroxide; 1.5mM/l EDTA and 1.5mM/l EGTA) was admixed respectively with solutions containing 0, 1, 10, 50, 100, 500 and 1000pM of a partially complementary oligonucleotide target sequence (gcccgttactgtcgtttttgtcc) in hybridisation buffer (10µl). The mixtures were incubated at 60°C for 60 min and allowed to equilibrate to room temperature prior to luminometry using the appropriate pH initiation solutions. The principle of these interactions is well known such that the complementary binding of the loop region to its target disrupts the stem binding causing the donor and acceptor moieties to become separated. Thus, in absence of target, the resulting mixture consisted of Structure A at pH 9 and Structure B at pH 12 (see Example 3). Increasing concentrations of target resulted in the formation of increasing amounts of Structure C, which is distinguishable from structure A on the basis of loss of quenching.
(C) 
Compound (d)-5'-CCGGTC CGGGCAATGACAGCAAAAACAGGGACCGGT-MR
GCCCGTTACTGTCGTTTTTGTCC
(MR = Methyl Red)

Thus, at pH 9, chemiluminescence intensity is proportional to the concentration of target sequence. By contrast, there is no relationship between chemiluminescence intensity and target concentration at pH 12 due to binding complex dissociation. This confirms that only acridinium esters capable of yielding chemi luminescence at moderate pH can be used in such assays.

### References

1. J. A. Miller, M. C. Coleman, R. S. Matthews, J. Org. Chem., 1993, 58, 2637-2639.
2. A. M. Holland, PhD thesis; Swansea University, 2002.
3. Z. Li, PhD Thesis, Swansea University, 1998.
4. L. Chen, PhD Thesis, Swansea University, 2000.
5. K. Smith, Z. Li, J. Yang, I. Weeks, J. S. Woodhead, J. Photochem. Photobiol. A: Chemistry, 2000, 132, 181-191.
6. T. Suzuki, S. Miyanari, Y. Tsubata, J. Org. Chem., 2001, 66, 216-224.
7. F. Menger, K. Caran, V. A. Seredyuk, Angew. Chem. Int. Ed., 2001, 40, 3905-3907.
8. B. F. L. M. Pattison, J. B. Stothers, R. G. Woolford, J. Am. Chem. Soc., 1956, 78, 2255-2256.
9. L. M. A. McNamara., M. J. I. Andrews, F. Mitzel, G. Siligardi, A. B. Tabor, J. Org. Chem., 2001, 66, 4585-4594.
10. Z. Li, Report 43, Swansea University.
11. S.E. Denmark; D. C. Forbes; D. S. Hays, J. Org. Chem., 1995, 60, 1391-1407.
12. C. D. Beard, K. Baum, J. Org. Chem., 1974, 39, 3875-3877. W. K. Fife, P. Ranganathan, M. Zeldin, J. Org. Chem., 1990, 55, 5610-5613.

## Claims

1. A compound of general formula (I) 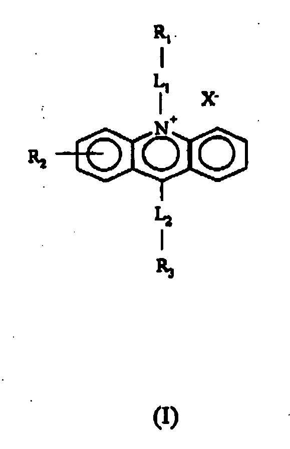 wherein:
either:
R₁ is a reactive group capable of reacting with an amine or thiol moiety;
L₁ is a hydrocarbon linker moiety comprising 2 - 12 carbon atoms, optionally substituted with hydroxy, halo, nitro or C₁-C₄ alkoxy; and R₂ is hydrogen, C₁-C₄ alkyl, C₁-C₄ haloalkyl, aryl, fused aryl, C₁-C₄ alkoxy, C₁-C₄ acyl, halide, hydroxy or nitro;
or, alternatively:
the combination R₁-L₁- comprises a C₁-C₄ alkyl group optionally substituted with hydroxy, halo, nitro or C₁-C₄ alkoxy; and
R₂ comprises a group R₄-L₁-, where R₄ is a reactive group capable of reacting with an amine or thiol moiety; and L₁ is as defined above;
L₂ is -C(=O)O-, -C(=O)-S- or -C(=O)N(SO₂R₅)-,
wherein, in each case, the -C(=O) is linked to the ring carbon atom, and
R₅ is C₁-C₈ alkyl, aryl, C₁-C₈ alkoxy or C₁-C₈ acyl;
R₃ is a phenyl group substituted independently at the 2 and 6 positions with nitro, fluorine, chlorine, bromine or trifluoromethyl groups, wherein at least one of the said substituents is electron-withdrawing such that the pKa of the conjugate acid of the leaving group formed from R₃ and the -0, -S or -N(SO₂R₅) of the L₂ group is ≤ about 9.5; and
X⁻ is an anion formed as the result of the synthesis and processing of the molecule;
wherein the compound may contain one or more additional R₂ moieties on either or both outer rings, provided that only one of said R₂ moieties may comprise an R₄-L₁- group;
thereby allowing production of chemiluminescent radiation on treatment with hydrogen peroxide in conditions maintained at about pH 6 to 10.

2. A compound as claimed in claim 1 wherein R₁, or R₄ is an active ester, a maleimide or an active halide.

3. A compound as claimed in claim 2 wherein R₁ or R₄ is a succinimidyl ester, an imidate ester, chlorocarbonyl, bromocarbonyl, iodocarbonyl, chlorosulfonyl or fluorodinitrophenyl.

4. A compound as claimed in claim 1 wherein, independently or in any combination:
R1 is an active ester, an imidate, or an active halide;
L₁ comprises 3 to 10 carbon atoms;
R₂ is hydrogen or C₁-C₄ alkyl;
L₂ is -C(=O)O-; and
X- is a halide or halide-containing anion.

5. A compound as claimed in claim 4 wherein, independently or in any combination:
R₁ is a succinimidyl ester, an imidate ester, chlorocarbonyl, bromocarbonyl, iodocarbonyl, chlorosulfonyl or fluorodinitrophenyl moiety;
L₁ comprises 3 to 10 methylene units;
R₂ is hydrogen; and
X⁻ is iodide, fluorosulfonate, trifluoromethanesulfonate or trifluoroacetate.

6. A compound as claimed in claim 1 wherein, independently or in any combination:
R₁ is a maleimide moiety;
L₁ comprises 3 to 10 methylene units;
R₂ is hydrogen; and
X⁻ is iodide, fluorosulfonate, trifluoromethanesulfonate or trifluoroacetate.

7. A compound as claimed in any one of claims 1 to 6 wherein R₃ is chosen such that the pKa of the conjugate acid of the L₂-R₃ leaving group is ≥ 3.

8. A compound according to any one of claims 1- 7 comprising:
9-(2,6-Bis(trifluoromethyl)phenoxycarbonyl)-10-(10-succinimidyloxycarbonyl decyl)acridinium trifluoromethanesulfonate;
9-(2,6-dibromophenoxycarbonyl)-10-(10-succinimidyloxycarbonyldecyl)acridinium\ trifluoromethanesulfonate;
9-(2,6-bis(triauoromemyl)phenoxycarbonyl)-10-(3-succinunidyloxycarbonyl propyl)acridinium trifluoromethanesulfonate;
9-(2,6-dibromophenoxycarbonyl)-10-(3-succinimidyloxycarbonylpropyl)acndinium trifluoromethanesulfonate;
9-(2,6-dinitrophenoxycarbonyl)-10-(10-succinimidyloxycarbonyldecyl) acridinium trifluoromethanesulfonate; or
9-(2,6-dinitrophenoxycarbonyl)-10-(3-succinimidyloxycubonylpropyl) acridinium trifluoromethanesulfonate.

9. A process for the preparation of a compound as claimed in any one of claims 1 to 8, the process comprising treating a compound of general formula (II): 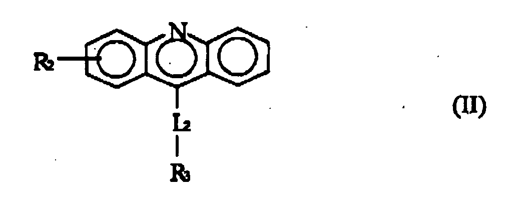 wherein R₂, L₂ md R₃ are as defined in general formula (I) with a compound of general formula (III)
X-L₁-R₁ (III)
wherein L₁ and R₁ are as defined in general formula (I) and X is a leaving group.

10. A method of detecting or monitoring the association or dissociation of a first and a second binding partner in a medium, wherein the first binding partner is labelled with a compound as claimed in any one of claims 1 to 8 and the second binding partner is labelled with a quencher or is itself a quencher, such that when the first and second binding partners are associated, the chemiluminescent signal of the compound as claimed in any one of claims 1 to 8 is modulated; the method comprising adding hydrogen peroxide to the medium while maintaining the pH at about 6-10 and detecting the emission of chemiluminescent radiation.

11. A method as claimed in claim 10, wherein the first and second binding partners are molecular species which associate or dissociate via a covalent reaction.

12. A method as claimed in claim 11, wherein the first and second binding partners are a ligand and an anti-ligand.

13. A method of detecting the presence or absence in a medium of an analyte comprising a quencher for a compound as claimed in any one of claims 1 to 8, the method comprising:
a) adding to the medium a specific binding partner for the analyte, said specific binding partner labelled with a compound as claimed in any one of claims 1 to 8 such that if analyte is present a specific binding complex is formed;
b) adding hydrogen peroxide to the medium while maintaining the pH at about 6-10; and
c) detecting the emission of chemiluminescent radiation, wherein a modulated signal of the compound as claimed in any one of claims 1 to 8 indicates the presence of the analyte.

14. A method of detecting the presence or absence in a medium of an analyte which is not a quencher for a compound as claimed in any one of claims 1 to 8, the method comprising:
a) adding to the medium a specific binding partner for the analyte, said specific binding partner labelled with a compound as claimed in any one of claims 1 to 8 or a quencher for a compound as claimed in any one of claims 1 to 8 such that if analyte is present a specific binding complex is formed;
b) adding to the medium simultaneously or sequentially a secondary binding reagent labelled with either a quencher for the compound as claimed in any one of claims I to 8 used in step (a) or a compound as claimed in any one of claims 1 to 8 if a quencher is used in step (a); and
c) detecting the presence of specific binding complex by a chemiluminescent method including the steps of adding hydrogen peroxide to the medium while maintaining the pH at about 6-10 and detecting the emission of chemiluminescent radiation, wherein a modulated signal of the compound as claimed in any one of claims 1 to 8 indicates the presence of the analyte.

15. A method of detecting the presence or absence in a medium of an analyte which is not a quencher for a compound as claimed in any one of claims 1 to 8, the method comprising:
a) adding to the medium a specific binding partner for the analyte labelled with a compound as claimed in any one of claims 1 to 8 or with a quencher for a compound as claimed in any one of claims 1 to 8 such that if analyte is present, a specific binding complex is formed;
b) adding to the medium simultaneously or sequentially a second specific binding partner labelled with either a quencher or a compound as claimed in any one of claims 1 to 8 as appropriate to the first labelled specific binding partner; and
c) detecting the presence of specific binding complex by a chemiluminescent method including the steps of adding hydrogen peroxide to the medium while maintaining the pH at about 6-10 and detecting the emission of chemiluminescent radiation, wherein a modulated signal of the compound as claimed in any one of claims 1 to 8 indicates the presence of the analyte.

16. A method for detecting the presence of inhibitors or promoters of a binding process, the method comprising mixing a first and a second specific binding partner labelled respectively with a compound as claimed in any one of claims 1 to 8 and a quencher, adding hydrogen peroxide to the medium while maintaining the pH at about 6-10 and detecting the emission of chemiluminescent radiation, wherein modulation of the chemiluminescent signal of the compound as claimed in any one of claims 1 to 8 indicates promotion of the binding process and the presence of an unmodulated signal indicates inhibition of the binding process.

17. A method as claimed in claim 16 for monitoring the cleavage of an enzyme substrate or the hybridisation of complementary nucleic acids.

18. A method as claimed in any one of claims 10 to 17 wherein the quencher is methyl red.

19. A reagent comprising a ligand or anti-ligand labelled with a compound as claimed in any one of claims 1 to 8.

20. A reagent as claimed in claim 19 wherein the ligand or anti-ligand is a nucleic acid, a peptide or a protein.

21. A reagent as claimed in claim 20 wherein the ligand or anti-ligand is further labelled with a quencher.

22. A reagent as claimed in claim 21 comprising a nucleic acid molecule comprising up to about 40 bases having ends which are mutually complementary and a central section containing a sequence of about 15 to 30 bases which is complementary to a target sequence and wherein the molecule is labelled at one end with a compound as claimed in any one of claims 1 to 8 and at the other end with a quencher.

23. A reagent as claimed in claim 19 which comprises a nucleic acid duplex each strand of which is labelled respectively with a compound as claimed in any one of claims 1 to 8 and a quencher such that attenuation of the light emission exists when the duplex is substantially intact but not when the duplex is rendered substantially non-intact by chemical or physical means.

24. A reagent as claimed in claim 21 comprising an enzyme substrate labelled at sites respectively either side of a cleavage site with a compound as claimed in any one of claims 1 to 8 and a quencher.

25. A reagent as claimed in any one of claims 22 to 24 wherein the quencher is methyl red.

26. A kit comprising a reagent as claimed in any one of claims 20 to 25, hydrogen peroxide solution and a buffer adapted to maintain the pH of the analyte containing medium at pH 6-10.

27. A kit as claimed in claim 26 further comprising a second reagent labelled with a quencher.

28. A kit as claimed in claim 27 wherein either:
the first reagent is a specific binding partner for an analyte and the second reagent is a secondary binding partner, or
the first reagent is a secondary binding partner and the second reagent is a specific binding partner for an analyte.

29. A kit as claimed in claim 27 or claim 28 wherein the quencher is methyl red.

30. A compound selected from the group consisting of:
9-(2,6-Bis(trifluoromethyl)phenoxycarbonyl)-10-(10-succinimidyloxycubonyldecyl) acridinium trifluoromethanesulfonate;
9-(2,6-dibromophenoxycarbonyl)-10-(10-succinimidyloxycarbonyldecyl)acridinium trifluoromethanesulfonate;
9-(2,6-bis(trifluoromethyl)phenoxycarbonyl)-10-(3-succinimidyloxycarbonyl propyl)acridinium trifluoromethanesulfonate;
9-(2,6-dibromophenoxycarbonyl)-10-(3-succinimidyloxycarbonylpropyl)acridinium trifluoromethanesulfonate;
9-(2,6-dinitrophenoxycarbonyl)-10-(10-succinimidylaxycarbonyldecyl)acridinium trifluoromethanesulfonate; and
9-(2,6-dinitrophenoxycarbonyl)-10-(3-succinimidyloxycarbonylpropyl)acidinium trifluoromethanesulfonate.

## Patentansprüche

1. Verbindung der allgemeinen Formel (I) 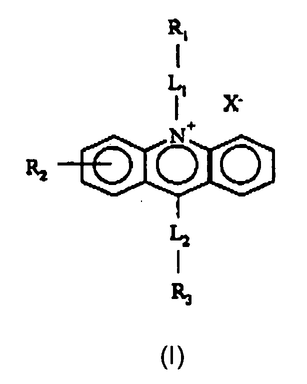 worin
entweder:
R₁ eine reaktive Gruppe ist, die mit einem Amin- oder Thiolrest reagieren kann,
L₁ ein Kohlenwasserstoffverknüpfungsgruppe-Rest ist, der 2 bis 12 Kohlenstoffatome umfasst und gegebenenfalls mit Hydroxy, Halogen, Nitro oder C₁-C₄-Alkoxy substituiert ist, und
R₂ Wasserstoff, C₁-C₄-Alkyl, C₁-C₄-Halogenalkyl, Aryl, anelliertes Aryl, C₁-C₄-Alkoxy, C₁-C₄-Acyl, Halogenid, Hydroxy oder Nitro ist,
oder alternativ:
die Kombination R₁-L₁- eine C₁-C₄-Alkylgruppe umfasst, die gegebenenfalls mit Hydroxy, Halogen, Nitro oder C₁-C₄-Alkoxy substituiert ist, und
R₂ eine Gruppe R₄-L₁- umfasst, bei der R₄ eine reaktive Gruppe ist, die mit
einem Amin- oder Thiolrest reagieren kann, und L₁ wie vorstehend definiert ist,
L₂ -C(=O)O-, -C(=O)-S- oder -C(=O)N(SO₂R₅)- ist,
wobei in jedem Fall das -C(=O) mit dem Ringkohlenstoffatom verknüpft ist und R₅ C₁-C₈-Alkyl, Aryl, C₁-C₈-Alkoxy oder C₁-C₈-Acyl ist,
R₃ eine Phenylgruppe ist, die unabhängig an der 2- und an der 6-Position mit Nitro-, Fluor-, Chlor-, Brom- oder Trifluormethylgruppen substituiert ist, wobei mindestens einer der Substituenten elektronenziehend ist, so dass der pKa der konjugierten Säure der Austrittsgruppe, die aus R₃ und dem -O, -S oder -N(SO₂R₅) der L₂-Gruppe gebildet ist, ≤ etwa 9,5 ist, und
X- ein Anion ist, das als das Ergebnis der Synthese und Verarbeitung des Moleküls gebildet worden ist,
wobei die Verbindung einen oder mehrere zusätzliche(n) R₂-Rest(e) an einem der äußeren Ringe oder beiden äußeren Ringen enthalten kann, mit der Maßgabe, dass nur einer der R₂-Reste eine R₄-L₁-Gruppe umfassen kann,
wodurch die Erzeugung von Chemilumineszenzstrahlung bei der Behandlung mit Wasserstoffperoxid bei Bedingungen, die bei etwa pH 6 bis 10 gehalten werden, ermöglicht wird.

2. Verbindung nach Anspruch 1, bei der R₁ oder R₄ ein aktiver Ester, ein Maleimid oder ein aktives Halogenid ist.

3. Verbindung nach Anspruch 2, bei der R₁ oder R₄ ein Succinimidylester, ein Imidatester, Chlorcarbonyl, Bromcarbonyl, lodcarbonyl, Chlorsulfonyl oder Fluordinitrophenyl ist.

4. Verbindung nach Anspruch 1, bei der unabhängig oder in jedweder Kombination:
R₁ ein aktiver Ester, ein Imidat oder ein aktives Halogenid ist,
L₁ 3 bis 10 Kohlenstoffatome umfasst,
R₂ Wasserstoff oder C₁-C₄-Alkyl ist,
L₂ -C(=O)O- ist und
X- ein Halogenid oder ein Halogenid-enthaltendes Anion ist.

5. Verbindung nach Anspruch 4, bei der unabhängig oder in jedweder Kombination:
R₁ ein Succinimidylester-, ein Imidatester-, Chlorcarbonyl-, Bromcarbonyl-, lodcarbonyl-, Chlorsulfonyl- oder Fluordinitrophenylrest ist;
L₁ 3 bis 10 Methyleneinheiten umfasst,
R₂ Wasserstoff ist und
X⁻ Iodid, Fluorsulfonat, Trifluormethansulfonat oder Trifluoracetat ist.

6. Verbindung nach Anspruch 1, bei der unabhängig oder in jedweder Kombination:
R₁ ein Maleimidrest ist,
L₁ 3 bis 10 Methyleneinheiten umfasst,
R₂ Wasserstoff ist und
X⁻ Iodid, Fluorsulfonat, Trifluormethansulfonat oder Trifluoracetat ist.

7. Verbindung nach einem der Ansprüche 1 bis 6, bei der R₃ so ausgewählt ist, dass der pKa der konjugierten Säure der L₂-R₃-Austrittsgruppe ≥3 ist.

8. Verbindung nach einem der Ansprüche 1 bis 7, umfassend:
9-(2,6-Bis(trifluormethyl)phenoxycarbonyl)-10-(10-succinimidyloxycarbonyldecyl)-acridiniumtrifluormethansulfonat,
9-(2,6-Dibromphenoxycarbonyl)-10-(10-succinimidyloxycarbonyldecyl)-acridiniumtrifluormethansulfonat,
9-(2,6-Bis(trifluormethyl)phenoxycarbonyl)-10-(3-succinimidyloxycarbonylpropyl)-acridiniumtrifluormethansulfonat,
9-(2,6-Dibromphenoxycarbonyl)-10-(3-succinimidyloxycarbonylpropyl)-acridiniumtrifluormethansulfonat,
9-(2,6-Dinitrophenoxycarbonyl)-10-(10-succinimidyloxycarbonyldecyl)-acridiniumtrifluormethansulfonat oder
9-(2,6-Dinitrophenoxycarbonyl)-10-(3-succinimidyloxycarbonylpropyl)-acridiniumtrifluormethansulfonat.

9. Verfahren zur Herstellung einer Verbindung nach einem der Ansprüche 1 bis 8, wobei das Verfahren das Behandeln einer Verbindung der allgemeinen Formel (II): 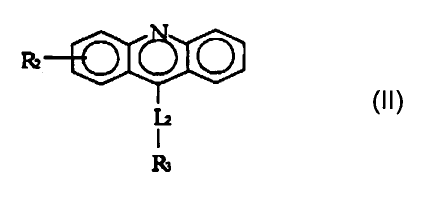 worin R₂, L₂ und R₃ wie bei der allgemeinen Formel (I) definiert sind, mit einer Verbindung der allgemeinen Formel (III)
X-L₁-R₁ (III)
worin L₁ und R₁ wie bei der allgemeinen Formel (I) definiert sind und X eine Austrittsgruppe ist, umfasst.

10. Verfahren zum Erfassen oder Überwachen der Assoziation oder Dissoziation eines ersten und eines zweiten Bindungspartners in einem Medium, wobei der erste Bindungspartner mit einer Verbindung nach einem der Ansprüche 1 bis 8 markiert ist und der zweite Bindungspartner mit einem Quencher markiert ist oder selbst ein Quencher ist, so dass dann, wenn der erste Bindungspartner und der zweite Bindungspartner assoziiert sind, das Chemilumineszenzsignal der Verbindung nach einem der Ansprüche 1 bis 8 moduliert wird, wobei das Verfahren das Zusetzen von Wasserstoffperoxid zu dem Medium, während der pH bei etwa 6 bis 10 gehalten wird, und das Erfassen der Emission von Chemilumineszenzstrahlung umfasst.

11. Verfahren nach Anspruch 10, bei dem der erste Bindungspartner und der zweite Bindungspartner molekulare Spezies sind, die mittels einer kovalenten Reaktion assoziieren oder dissoziieren.

12. Verfahren nach Anspruch 11, bei dem der erste Bindungspartner und der zweite Bindungspartner ein Ligand und ein Anti-Ligand sind.

13. Verfahren zum Erfassen der Gegenwart oder der Abwesenheit eines Analyten, der einen Quencher für eine Verbindung nach einem der Ansprüche 1 bis 8 umfasst, in einem Medium, wobei das Verfahren umfasst:
a) Zusetzen eines spezifischen Bindungspartners für den Analyten zu dem Medium, wobei der spezifische Bindungspartner mit einer Verbindung nach einem der Ansprüche 1 bis 8 markiert ist, so dass dann, wenn der Analyt vorliegt, ein spezifischer Bindungskomplex gebildet wird,
b) Zusetzen von Wasserstoffperoxid zu dem Medium, während der pH bei etwa 6 bis 10 gehalten wird, und
c) Erfassen der Emission von Chemilumineszenzstrahlung, wobei ein moduliertes Signal der Verbindung nach einem der Ansprüche 1 bis 8 die Gegenwart des Analyten anzeigt.

14. Verfahren zum Erfassen der Gegenwart oder der Abwesenheit eines Analyten, der kein Quencher für eine Verbindung nach einem der Ansprüche 1 bis 8 ist, in einem Medium, wobei das Verfahren umfasst:
a) Zusetzen eines spezifischen Bindungspartners für den Analyten zu dem Medium, wobei der spezifische Bindungspartner mit einer Verbindung nach einem der Ansprüche 1 bis 8 oder einem Quencher für eine Verbindung nach einem der Ansprüche 1 bis 8 markiert ist, so dass dann, wenn der Analyt vorliegt, ein spezifischer Bindungskomplex gebildet wird,
b) gleichzeitiges oder aufeinander folgendes Zusetzen eines sekundären Bindungsreagenzes, das entweder mit einem Quencher für die Verbindung nach einem der Ansprüche 1 bis 8, der im Schritt (a) verwendet worden ist, oder einer Verbindung nach einem der Ansprüche 1 bis 8, wenn im Schritt (a) ein Quencher verwendet wird, markiert ist, zu dem Medium, und
c) Erfassen der Gegenwart eines spezifischen Bindungskomplexes durch ein Chemilumineszenzverfahren, umfassend die Schritte des Zusetzens von Wasserstoffperoxid zu dem Medium, während der pH bei etwa 6 bis 10 gehalten wird, und des Erfassens der Emission von Chemilumineszenzstrahlung, wobei ein moduliertes Signal der Verbindung nach einem der Ansprüche 1 bis 8 die Gegenwart des Analyten anzeigt.

15. Verfahren zum Erfassen der Gegenwart oder der Abwesenheit eines Analyten, der kein Quencher für eine Verbindung nach einem der Ansprüche 1 bis 8 ist, in einem Medium, wobei das Verfahren umfasst:
a) Zusetzen eines spezifischen Bindungspartners für den Analyten, der mit einer Verbindung nach einem der Ansprüche 1 bis 8 oder einem Quencher für eine Verbindung nach einem der Ansprüche 1 bis 8 markiert ist, zu dem Medium, so dass dann, wenn der Analyt vorliegt, ein spezifischer Bindungskomplex gebildet wird,
b) gleichzeitiges oder aufeinander folgendes Zusetzen eines zweiten spezifischen Bindungspartners, der entweder mit einem Quencher oder einer Verbindung nach einem der Ansprüche 1 bis 8 gemäß dem ersten markierten spezifischen Bindungspartner markiert ist, zu dem Medium, und
c) Erfassen der Gegenwart eines spezifischen Bindungskomplexes durch ein Chemilumineszenzverfahren, umfassend die Schritte des Zusetzens von Wasserstoffperoxid zu dem Medium, während der pH bei etwa 6 bis 10 gehalten wird, und des Erfassens der Emission von Chemilumineszenzstrahlung, wobei ein moduliertes Signal der Verbindung nach einem der Ansprüche 1 bis 8 die Gegenwart des Analyten anzeigt.

16. Verfahren zum Erfassen der Gegenwart von Inhibitoren oder Promotoren eines Bindungsvorgangs, wobei das Verfahren das Mischen eines ersten spezifischen Bindungspartners und eines zweiten spezifischen Bindungspartners, die mit einer Verbindung nach einem der Ansprüche 1 bis 8 bzw. einem Quencher markiert sind, das Zusetzen von Wasserstoffperoxid zu dem Medium, während der pH bei etwa 6 bis 10 gehalten wird, und das Erfassen der Emission von Chemilumineszenzstrahlung umfasst, wobei die Modulierung des Chemilumineszenzsignals der Verbindung nach einem der Ansprüche 1 bis 8 die Förderung des Bindungsvorgangs anzeigt und die Gegenwart eines unmodulierten Signals die Inhibierung des Bindungsvorgangs anzeigt.

17. Verfahren nach Anspruch 16 zum Überwachen der Spaltung eines Enzymsubstrats oder der Hybridisierung von komplementären Nukleinsäuren.

18. Verfahren nach einem der Ansprüche 10 bis 17, bei dem der Quencher Methylrot ist.

19. Reagenz, das einen Liganden oder einen Anti-Liganden umfasst, der mit einer Verbindung nach einem der Ansprüche 1 bis 8 markiert ist.

20. Reagenz nach Anspruch 19, bei dem der Ligand oder der Anti-Ligand eine Nukleinsäure, ein Peptid oder ein Protein ist.

21. Reagenz nach Anspruch 20, bei dem der Ligand oder der Anti-Ligand ferner mit einem Quencher markiert ist.

22. Reagenz nach Anspruch 21, das ein Nukleinsäuremolekül umfasst, das bis zu etwa 40 Basen mit Enden, die zueinander komplementär sind, und einen zentralen Abschnitt umfasst, der eine Sequenz von etwa 15 bis 30 Basen umfasst, die zu einer Zielsequenz komplementär ist, und wobei das Molekül an einem Ende mit einer Verbindung nach einem der Ansprüche 1 bis 8 und an dem anderen Ende mit einem Quencher markiert ist.

23. Reagenz nach Anspruch 19, das einen Nukleinsäureduplex umfasst, bei dem jeder Strang jeweils mit einer Verbindung nach einem der Ansprüche 1 bis 8 und einem Quencher markiert ist, so dass eine Dämpfung der Lichtemission vorliegt, wenn der Duplex im Wesentlichen intakt ist, jedoch nicht vorliegt, wenn der Duplex durch chemische oder physikalische Mittel im Wesentlichen nicht-intakt gemacht wird.

24. Reagenz nach Anspruch 21, das ein Enzymsubstrat umfasst, das an Stellen an beiden Seiten einer Spaltstelle mit einer Verbindung nach einem der Ansprüche 1 bis 8 bzw. einem Quencher markiert ist.

25. Reagenz nach einem der Ansprüche 22 bis 24, bei dem der Quencher Methylrot ist.

26. Kit, das ein Reagenz nach einem der Ansprüche 20 bis 25, eine Wasserstoffperoxidlösung und einen Puffer, der angepasst ist, den pH des Analytenthaltenden Mediums bei pH 6 bis 10 zu halten, umfasst.

27. Kit nach Anspruch 26, das ferner ein zweites Reagenz umfasst, das mit einem Quencher markiert ist.

28. Kit nach Anspruch 27, bei dem entweder
das erste Reagenz ein spezifischer Bindungspartner für einen Analyten und das zweite Reagenz ein sekundärer Bindungspartner ist, oder
das erste Reagenz ein sekundärer Bindungspartner und das zweite Reagenz ein spezifischer Bindungspartner für einen Analyten ist.

29. Kit nach Anspruch 27 oder Anspruch 28, bei dem der Quencher Methylrot ist.

30. Verbindung, ausgewählt aus der Gruppe, bestehend aus:
9-(2,6-Bis(trifluormethyl)phenoxycarbonyl)-10-(10-succinimidyloxycarbonyldecyl)-acridiniumtrifluormethansulfonat,
9-(2,6-Dibromphenoxycarbonyl)-10-(10-succinimidyloxycarbonyldecyl)-acridiniumtrifluormethansulfonat,
9-(2,6-Bis(trifluormethyl)phenoxycarbonyl)-10-(3-succinimidyloxycarbonylpropyl)-acridiniumtrifluormethansulfonat,
9-(2,6-Dibromphenoxycarbonyl)-10-(3-succinimidyloxycarbonylpropyl)-acridiniumtrifluormethansulfonat,
9-(2,6-Dinitrophenoxycarbonyl)-10-(10-succinimidyloxycarbonyldecyl)-acridiniumtrifluormethansulfonat und
9-(2,6-Dinitrophenoxycarbonyl)-10-(3-succinimidyloxycarbonylpropyl)-acridiniumtrifluormethansulfonat.

## Revendications

1. Composé représenté par la formule générale (I) : 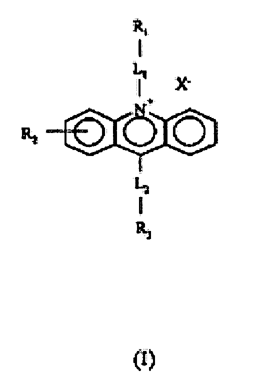 dans laquelle :
soit :
- R₁ représente un groupe réactif capable de réagir avec une fraction amine ou thiol ;
- L₁ représente une fraction de liaison hydrocarbonée comprenant 2-12 atomes de carbone, facultativement substituée par hydroxy, halo, nitro ou alcoxy en C₁-C₄ ; et
- R₂ représente hydrogène, alkyle en C₁-C₄, haloalkyle en C₁-C₄, aryle, aryle fusionné, alcoxy en C₁-C₄, acyle en C₁-C₄, halogénure, hydroxy ou nitro ;
soit, en variante :
- la combinaison R₁-L₁- comprend un groupe alkyle en C₁-C₄ facultativement substitué par hydroxy, halo, nitro ou alcoxy en C₁-C₄ ; et
- R₂ comprend un groupe R₄-L₁-, où R₄ est un groupe réactif capable de réagir avec une fraction amine ou thiol ; et L₁ est tel que défini ci-dessus ;
- L₂ représente -C(=O)O-, -C(=O)-S- ou -C(=O)N(SO₂R₅)-, où, dans chaque cas, le -C(=O) est lié à l'atome de carbone de cycle, et R₅ représente alkyle en C₁-C₈, aryle, alcoxy en C₁-C₈ ou acyle en C₁-C₈ ;
- R₃ représente un groupe phényle substitué indépendamment dans les positions 2 et 6 par nitro, fluor, chlore, brome ou des groupes trifluorométhyle, où au moins l'un desdits substituants est attracteur d'électrons de telle sorte que le pKa de l'acide conjugué du groupe partant formé à partir de R₃ et du -O, -S ou -N(SO₂R₅) du groupe L₂ est ≤ environ 9,5 ; et
- X⁻ est un anion formé comme résultat de la synthèse et du traitement de la molécule ;
le composé pouvant contenir une ou plusieurs fractions R₂ supplémentaires sur l'un ou l'autre des deux cycles externes ou sur les deux, à la condition que seule l'une desdites fractions R₂ puisse comprendre un groupe R₄-L₁-, permettant ainsi la production d'un rayonnement chimiluminescent lors d'un traitement par le peroxyde d'hydrogène dans des conditions maintenues à un pH d'environ 6 à 10.

2. Composé selon la revendication 1, dans lequel R₁ ou R₄ est un ester actif, un maléimide ou un halogénure actif.

3. Composé selon la revendication 2, dans lequel R₁ ou R₄ représente un ester succinimidylique, un ester imidate, chlorocarbonyle, bromocarbonyle, iodocarbonyle, chlorosulfonyle ou fluorodinitrophényle.

4. Composé selon la revendication 1, dans lequel, indépendamment ou dans n'importe quelle combinaison :
- R₁ est un ester actif, un imidate ou un halogénure actif ;
- L₁ comprend de 3 à 10 atomes de carbone ;
- R₂ représente hydrogène ou alkyle en C₁-C₄ ;
- L₂ représente -C(=O)O- ; et
- X⁻ est un halogénure ou un anion contenant un halogénure.

5. Composé selon la revendication 4, dans lequel, indépendamment ou dans n'importe quelle combinaison :
- R₁ représente un ester succinimidylique, un ester imidate, une fraction chlorocarbonyle, bromocarbonyle, iodocarbonyle, chlorosulfonyle ou fluorodinitrophényle ;
- L₁ comprend de 3 à 10 unités méthylène ;
- R₂ représente hydrogène ; et
- X⁻ représente iodure, fluorosulfonante, trifluorométhanesulfonate ou trifluoroacétate.

6. Composé selon la revendication 1, dans lequel, indépendamment ou dans n'importe quelle combinaison :
- R₁ est une fraction maléimide ;
- L₁ comprend de 3 à 10 unités méthylène ;
- R₂ représente hydrogène ; et
- X⁻ représente iodure, fluorosulfonate, trifluorométhanesulfonate ou trifluoroacétate.

7. Composé selon l'une quelconque des revendications 1 à 6, dans lequel R₃ est choisi de telle sorte que le pKa de l'acide conjugué du groupe partant L₂-R₃ est ≥ 3.

8. Composé selon l'une quelconque des revendications 1 à 7, comprenant :
- le trifluorométhanesulfonate de 9-(2,6-bis(trifluorométhyl)phénoxycarbonyl)-10-(10-succinimidyloxycarbonyldécyl)acridinium ;
- le trifluorométhanesulfonate de 9-(2,6-dibromophénoxycarbonyl)-10-(10-succinimidyloxycarbonyldécyl)acridinium ;
- le trifluorométhanesulfonate de 9-(2,6-bis(trifluorométhyl)phénoxycarbonyl)-10-(3-succinimidyloxycarbonylpropyl)acridinium ;
- le trifluorométhanesulfonate de 9-(2,6-dibromophénoxycarbonyl)-10-(3-succinimidyloxycarbonylpropyl)acridinium ;
- le trifluorométhanesulfonate de 9-(2,6-dinitrophénoxycarbonyl)-10-(10-succinimidyloxycarbonyldécyl)acridinium ; ou
- le trifluorométhanesulfonate de 9-(2,6-dinitrophénoxycarbonyl)-10-(3-succinimidyloxycarbonylpropyl)acridinium.

9. Procédé de préparation d'un composé selon l'une quelconque des revendications 1 à 8, le procédé comprenant le traitement d'un composé représenté par la formule générale (II) : 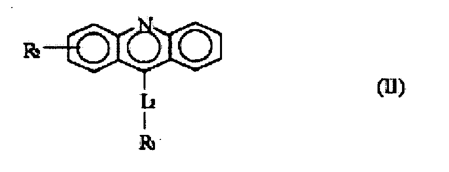 dans laquelle R₂, L₂ et R₃ sont tels que définis dans la formule générale (I), avec un composé représenté par la formule générale (III) :
X-L₁-R₁ (III)
dans laquelle L₁ et R₁ sont tels que définis dans la formule générale (I) et X est un groupe partant.

10. Procédé de détection ou de surveillance de l'association ou de la dissociation d'un premier et d'un second partenaire de liaison dans un milieu, le premier partenaire de liaison étant marqué par un composé selon l'une quelconque des revendications 1 à 8 et le second partenaire de liaison étant marqué par un désactivateur ou étant lui-même un désactivateur, de telle sorte que, lorsque les premier et second partenaires de liaison sont associés, le signal chimiluminescent du composé selon l'une quelconque des revendications 1 à 8 est modulé, le procédé comprenant l'addition de peroxyde d'hydrogène au milieu tout en maintenant le pH à environ 6-10, et la détection de l'émission de rayonnement chimiluminescent.

11. Procédé selon la revendication 10, dans lequel les premier et second partenaires de liaison sont des espèces moléculaires qui s'associent ou se dissocient par l'intermédiaire d'une réaction covalente.

12. Procédé selon la revendication 11, dans lequel les premier et second partenaires de liaison sont un ligand et un anti-ligand.

13. Procédé de détection de la présence ou de l'absence dans un milieu d'un analyte comprenant un désactivateur pour un composé selon l'une quelconque des revendications 1 à 8, le procédé comprenant les opérations consistant à :
a) ajouter au milieu un partenaire de liaison spécifique pour l'analyte, ledit partenaire de liaison spécifique étant marqué par un composé selon l'une quelconque des revendications 1 à 8, de telle sorte que si l'analyte est présent, un complexe de liaison spécifique est formé ;
b) ajouter du peroxyde d'hydrogène au milieu tout en maintenant le pH à environ 6-10 ; et
c) détecter l'émission de rayonnement chimiluminescent, un signal modulé du composé selon l'une quelconque des revendications 1 à 8 indiquant la présence de l'analyte.

14. Procédé de détection de la présence ou de l'absence dans un milieu d'un analyte qui n'est pas un désactivateur pour un composé selon l'une quelconque des revendications 1 à 8, le procédé comprenant les opérations consistant à :
a) ajouter au milieu un partenaire de liaison spécifique pour l'analyte, ledit partenaire de liaison spécifique étant marqué par un composé selon l'une quelconque des revendications 1 à 8 ou un désactivateur pour un composé selon l'une quelconque des revendications 1 à 8, de telle sorte que, si l'analyte est présent, un complexe de liaison spécifique est formé ;
b) ajouter au milieu, simultanément ou séquentiellement, un réactif de liaison secondaire marqué par soit un désactivateur pour un composé selon l'une quelconque des revendications 1 à 8 utilisé dans l'étape (a), soit par un composé selon l'une quelconque des revendications 1 à 8 si un désactivateur est utilisé dans l'étape (a) ; et
c) détecter la présence de complexe de liaison spécifique par un procédé chimiluminescent, comprenant les étapes d'addition de peroxyde d'hydrogène au milieu tout en maintenant le pH à environ 6-10 et de détection de l'émission de rayonnement chimiluminescent, un signal modulé du composé selon l'une quelconque des revendications 1 à 8 indiquant la présence de l'analyte.

15. Procédé de détection de la présence ou de l'absence dans un milieu d'un analyte qui n'est pas un désactivateur pour un composé selon l'une quelconque des revendications 1 à 8, le procédé comprenant les opérations consistant à :
a) ajouter au milieu un partenaire de liaison spécifique pour l'analyte marqué par un composé selon l'une quelconque des revendications 1 à 8 ou par un désactivateur pour un composé selon l'une quelconque des revendications 1 à 8, de telle sorte que si l'analyte est présent, un complexe de liaison spécifique est formé ;
b) ajouter au milieu, simultanément ou séquentiellement, un second partenaire de liaison spécifique marqué soit par un désactivateur, soit par un composé selon l'une quelconque des revendications 1 à 8 tel qu'approprié au premier partenaire de liaison spécifique marqué ; et
c) détecter la présence de complexe de liaison spécifique par un procédé chimiluminescent, comprenant les étapes d'addition de peroxyde d'hydrogène au milieu tout en maintenant le pH à environ 6-10 et de détection de l'émission de rayonnement chimiluminescent, un signal modulé du composé selon l'une quelconque des revendications 1 à 8 indiquant la présence de l'analyte.

16. Procédé de détection de la présence d'inhibiteurs ou de promoteurs d'un procédé de liaison, le procédé comprenant les opérations consistant à mélanger un premier et un second partenaire de liaison spécifique marqués respectivement par un composé selon l'une quelconque des revendications 1 à 8 et un désactivateur, à ajouter du peroxyde d'hydrogène au milieu tout en maintenant le pH à environ 6-10, et à détecter l'émission de rayonnement chimiluminescent, une modulation du signal chimiluminescent du composé selon l'une quelconque des revendications 1 à 8 indiquant une activation du procédé de liaison et la présence d'un signal non modulé indiquant une inhibition du procédé de liaison.

17. Procédé selon la revendication 16 pour surveiller le clivage d'un substrat d'enzyme ou l'hybridation d'acides nucléiques complémentaires.

18. Procédé selon l'une quelconque des revendications 10 à 17, dans lequel le désactivateur est du rouge de méthyle.

19. Réactif comprenant un ligand ou un anti-ligand marqué par un composé selon l'une quelconque des revendications 1 à 8.

20. Réactif selon la revendication 19, dans lequel le ligand ou l'anti-ligand est un acide nucléique, un peptide ou une protéine.

21. Réactif selon la revendication 20, dans lequel le ligand ou l'anti-ligand est encore marqué par un désactivateur.

22. Réactif selon la revendication 21 comprenant une molécule d'acide nucléique comprenant jusqu'à environ 40 bases ayant des extrémités qui sont mutuellement complémentaires et une section centrale contenant une séquence d'environ 15 à 30 bases qui est complémentaire à une séquence cible et dans lequel la molécule est marquée à une extrémité par un composé selon l'une quelconque des revendications 1 à 8 et à l'autre extrémité par un désactivateur.

23. Réactif selon la revendication 19 qui comprend un duplex d'acide nucléique dont chaque brin est marqué respectivement par un composé selon l'une quelconque des revendications 1 à 8 et un désactivateur, de telle sorte qu'une atténuation de l'émission de lumière existe lorsque le duplex d'acide nucléique est sensiblement intact, mais n'existe pas lorsque le duplex est rendu sensiblement non-intact par des moyens chimiques ou physiques.

24. Réactif selon la revendication 21 comprenant un susbtrat d'enzyme marqué à des sites respectivement de l'un ou l'autre côté d'un site de clivage par un composé selon l'une quelconque des revendications 1 à 8 et un désactivateur.

25. Réactif selon l'une quelconque des revendications 22 à 24, dans lequel le désactivateur est le rouge de méthyle.

26. Coffret comprenant un réactif selon l'une quelconque des revendications 20 à 25, une solution de peroxyde d'hydrogène et un tampon apte à maintenir le pH du milieu contenant l'analyte à un pH de 6-10.

27. Coffret selon la revendication 26 comprenant également un second réactif marqué par un désactivateur.

28. Coffret selon la revendication 27 dans lequel :
- soit le premier réactif est un partenaire de liaison spécifique pour un analyte et le second réactif est un partenaire de liaison secondaire ;
- soit le premier réactif est un partenaire de liaison secondaire et le second réactif est un partenaire de liaison spécifique pour un analyte.

29. Coffret selon l'une des revendications 27 ou 28, dans lequel le désactivateur est le rouge de méthyle.

30. Composé choisi dans le groupe constitué par :
- le trifluorométhanesulfonate de 9-(2,6-bis(trifluorométhyl)phénoxycarbonyl)-10-(10-succinimidyloxycarbonyldécyl)acridinium ;
- le trifluorométhanesulfonate de 9-(2,6-dibromophénoxycarbonyl)-10-(10-succinimidyloxycarbonyldécyl)acridinium ;
- le trifluorométhanesulfonate de 9-(2,6-bis(trifluorométhyl)phénoxycarbonyl)-10-(3-succinimidyloxycarbonylpropyl)acridinium ;
- le trifluorométhanesulfonate de 9-(2,6-dibromophénoxycarbonyl)-10-(3-succinimidyloxycarbonylpropyl)acridinium ;
- le trifluorométhanesulfonate de 9-(2,6-dinitrophénoxycarbonyl)-10-(10-succinimidyloxycarbonylpropyl)acridinium ; et
- le trifluorométhanesulfonate de 9-(2,6-dinitrophénoxycarbonyl)-10-(3-succinimidyloxycarbonylpropyl)acridinium.
